# EUROPÄISCHE PATENTSCHRIFT

(11) **EP 3 115 039 B1**
(45) Veröffentlichungstag und Bekanntmachung des Hinweises auf die Patenterteilung: **15.12.2021**
(21) Anmeldenummer: 15176005.5
(22) Anmeldetag: 09.07.2015
(51) Int. Cl.: A61K 9/127, A61K 38/00, A61K 31/7105, A61K 31/711

(54) **VERFAHREN ZUM HERSTELLEN EINER FUSIONSMISCHUNG ZUR ÜBERTRAGUNG EINES GELADENEN MOLEKÜLS IN UND/ODER DURCH EINE LIPIDMEMBRAN**
METHOD FOR PRODUCING A FUSION MIXTURE FOR TRANSFERRING A CHARGED MOLECULE INTO AND/OR THROUGH A LIPID MEMBRANE
PROCEDE DE FABRICATION D'UN MELANGE EN FUSION DESTINE AU TRANSFERT D'UNE MOLECULE CHARGEE DANS ET/OU A TRAVERS UNE MEMBRANE DE LIPIDE

(43) Veröffentlichungstag der Anmeldung: 11.01.2017
(73) Patentinhaber: beniag GmbH, 52428 Jülich (DE); Hoffmann, Marco, 41812 Erkelenz/Lövenich (DE)
(72) Erfinder: Hoffmann, Bernd, 52428 Jülich (DE); Csiszar, Agnes, 52428 Jülich (DE); Hersch, Nils, 41812 Venrath (DE); Zantl, Roman, 85598 Flurweg 5a (DE); Merkel, Rudolf, 52428 Jülich (DE); Hoffmann, Marco, 41849 Wassenberg (DE)
(74) Vertreter: Grünecker Patent- und Rechtsanwälte PartG mbB

(56) Entgegenhaltungen:
- WO-A1-93/00888
- WO-A2-2011/003406
- US-A- 5 227 170
- AGNES CSISZÁR ET AL: "Novel Fusogenic Liposomes for Fluorescent Cell Labeling and Membrane Modification", BIOCONJUGATE CHEMISTRY, Bd. 21, Nr. 3, 17. März 2010 (2010-03-17), Seiten 537-543, XP055137866, ISSN: 1043-1802, DOI: 10.1021/bc900470y
- YOSHIKAWA T ET AL: "Augmentation of antigen-specific immune responses using DNA-fusogenic liposome vaccine", BIOCHEMICAL AND BIOPHYSICAL RESEARCH COMMUNICATIONS, ACADEMIC PRESS INC. ORLANDO, FL, US, Bd. 325, Nr. 2, 10. Dezember 2004 (2004-12-10), Seiten 500-505, XP004626821, ISSN: 0006-291X, DOI: 10.1016/J.BBRC.2004.10.056
- ZHANG HONG-WEI ET AL: "Successful transfection of hepatoma cells after encapsulation of plasmid DNA into negatively charged liposomes", BIOTECHNOLOGY AND BIOENGINEERING, Bd. 96, Nr. 1, Januar 2007 (2007-01), Seiten 118-124, XP002745625, ISSN: 0006-3592
- Ulla Wienhues ET AL: "A Novel Method for Transfection and Expression of Reconstituted DNA-Protein Complexes in Eukaryotic Cells", DNA, vol. 6, no. 1, 1 February 1987 (1987-02-01), pages 81-89, XP055501259, US ISSN: 0198-0238, DOI: 10.1089/dna.1987.6.81

## Beschreibung

Die Erfindung betrifft ein Verfahren zum Herstellen einer Fusionsmischung für eine Übertragung eines geladenen Moleküls in und/oder durch eine Lipidmembran sowie ein Verfahren zur Übertragung eines geladenen Moleküls in und/oder durch eine Lipidmembran mittels Fusion.

Für unterschiedlichste Anwendungen beispielsweise im Bereich der Grundlagenforschung, Biotechnologie oder auch Therapie, besteht ein Bedarf an Systemen, mit deren Hilfe geladene Moleküle, wie beispielsweise DNA oder unterschiedliche RNA-Moleküle in lebende Zellen übertragen werden.

Zwei nicht-virale Hauptübertragungsmechanismen für geladene Moleküle sind bekannt und werden routinemäßig eingesetzt. Hierbei handelt es sich für den Fall von Nukleinsäuren um die Übertragung mittels Endozytose (Lipofektion) sowie mittels Elektroporation.

Aus Khalil et al. (I.A. Khalil, K. Kogure, H. Akita, H. Harashima, 2006. Uptake Pathways and Subsequent Intracellular Trafficking in Nonviral Gene Delivery. PHARMACOLOGICAL RE-VIEWS, Vol. 58, No. 1, 32-45) ist bekannt, zwischen endozytotischen und nicht endozytotischen Aufnahmeverfahren für DNA zu unterscheiden. In der wissenschaftlichen Gemeinschaft wird akzeptiert, dass der Hauptweg der Aufnahme von DNA mittels Lipoplexen (auch kationische Lipid-DNA-Komplexe genannt) unter Normalbedingungen auf dem endozytotischen Wege erfolgt. Als Endozytose wird die vesikuläre Aufnahme extrazellulärer Makromoleküle bezeichnet, die nachfolgend enzymatisch in Lysosomen abgebaut und für eigene Stoffwechselprozesse eingesetzt werden. Hierdurch gehen im Allgemeinen die funktionellen Eigenschaften der aufgenommenen Makromoleküle vollständig verloren. Die bis Mitte der 1990er Jahre postulierte direkte Aufnahme von Makromolekülen nach der Anhaftung der Lipide der Lipoplexe mit der leicht negativ geladenen Zellmembran ist daher nicht haltbar, sondern allenfalls in sehr geringem Anteil beteiligt.

Dies wird auch durch die Arbeit von Friend et al. belegt, bei der DNA in intrazellulären Einschlüssen über elektronenmikroskopische Aufnahmen gezeigt wurden, die nur durch Endozytose entstanden sein konnten (Friend DS, Papahadjopoulos D, and Debs RJ (1996) Endocytosis and intracellular processing accompanying transfection mediated by cationic liposomes. Biochim Biophys Acta 1278:41―50). Dieser Aufnahmeweg wird auch durch Weijun und Szoka Jr. bestätigt (Weijun Li and Francis C. Szoka Jr. 2007. Lipid-based Nanoparticles for Nucleic Acid Delivery. Pharmaceutical Research, 24, 438-449.).

Eine Alternative zum endozytotischen Aufnahmemechanismus ist die Elektroporation. Hierbei werden ebenso wie beim endosomalen Aufnahmemechanismus in einem ersten Schritt Lipoplexe gebildet, die aufgrund ihrer Ladung eine erste Bindung zur Zelloberfläche aufbauen. Danach wird durch Anlegung eines kurzzeitig angelegten Spannungspulses die Zellmembran derart destabilisiert, dass mittels temporärer Porenbildung Lipoplexe in das Zellinnere, das sogenannte Cytoplasma der Zelle gelangen. Elektroporation nutzt das Phänomen der Destabilisierung von Lipidmembranen, wenn ihr natürlicher elektrischer Widerstand durch Anlegung eines elektrischen Pulses im Nano- bis Mikrosekundenbereich aufgehoben wird (Tsong, Biophysical Journal, 1991 60:297-306, Electroporation of cell membranes; Weaver, Journal of Cell Biochemistry, 1993, 51:426-435, Electroporation: a general phenomenon for manipulating cells and tissues). Gebildete Poren in der Zellmembran bleiben für einige Millisekunden erhalten und erlauben mittels passiver Diffusion einen rapiden Molekülaustausch zwischen Innerem der Zelle und dem umgebenden Medium.

Zum Aufbau der für den endosomalen als auch nicht-endosomalen Übertragungsmechanismus notwendigen Lipoplexe werden zu bestimmten Anteilen immer kationische Lipide bzw. in Wechselwirkung mit multivalenten Kationen positiv geladene Lipide verwendet, die aufgrund von Ladungswechselwirkungen mit negativ geladenen Nukleinsäuren hochmolekulare Lipoplexe aufbauen. Derartige Lipoplexe können unterschiedlichste Konformationen bzw. Lipidphasen aufbauen (Tresset, PMC Biophysics, 2009, 2:3, The multiple faces of selfassembled lipidic systems) wobei der Anteil an positiv geladenen Lipiden immer hoch ist. Da kationische Lipide in biologischen Systemen nahezu nicht vorkommen, induzieren diese nach Einbringung in lebende Systeme eine Reihe toxischer Effekte bis hin zur Letalität, die mit steigender Menge der entsprechende Lipide ansteigen (Dass, Journal of Molecular Medicine, 2004, 82:579-591, Lipoplex-mediated delivery of nucleic acids: factors affecting in vivo transfection).

Es ist bekannt, dass die Ausbildung kationischer Liposomen aus den Ausgangsbestandteilen schwierig zu kontrollieren ist, da unterschiedliche Strukturen aus den Ausgangssubstanzen ausgebildet werden können. Kationische Lipide werden daher zur Ausbildung von Liposomen vorab regelmäßig mit neutralen Lipiden als sogenannte Helferlipide gemischt, da kationische Lipide allein die Liposomenausbildung offensichtlich nicht gewährleisten können. Als Helfer wird z. B. DOPE oder Cholesterol verwendet, wie dies aus der oben genannten Literatur Khalil et al. bekannt ist.

Als weitere Methodik zur Übertragung von Molekülen ist die Fusion fusogener Liposomen mit Lipidmembranen bekannt. Basierend auf identischen Lipidsorten wie oben für positiv geladene sowie neutrale Lipide beschrieben, besitzen derartige fusogene Liposome mindestens eine weitere dritte Komponente, bei der es sich um ein aromatisches Lipid handelt (Csiszár A. et al. Novel Fusogenic Liposomes for Fluorescent Cell Labeling and Membrane Modification, Bioconjugate Chemistry, 2010, 21, 537-543; Kleusch Ch. et al. Fluorescent lipids: functional parts of fusogenic liposomes and tools for cell membrane labeling and visualization, Molecules, 2011, 16, 221-250). Hier erfolgt eine Fusion der Liposomen mit einer weiteren Lipidmembran, beispielsweise einer Zellmembran in einem Verhältnis von 1: 0,1: 1 (positives Lipid : aromatische Komponente : neutrales Lipid). Mögliche Mischungsverhältnisse für eine Fusion werden auch in der WO 2011/003406 und der WO 2014/154203 beschrieben.

Eine Übertragung geladener Moleküle mittels fusogener Liposomen ist schwierig und unzureichend effizient, da die geladenen Moleküle die Ladungsdichte der positiven Lipide reduzieren (partielle Neutralisation). Da fusogene Liposomen hauptsächlich einfache lipidummantelte Systeme darstellen, an oder in die Moleküle von Interesse eingebracht sind, ist die Konzentration kationischer Lipide deutlich niedriger als bei klassischen Lipoplex-Übertragungsmechanismen. Zusätzlich werden derartige Lipide nur in die Plasmamembran eingeschleust und nicht direkt auch in das Lumen der Zelle transportiert.

Da elektrostatische Wechselwirkungen des Übertragungssystems mit der Zielmembran für alle oben beschriebenen Techniken von großer Wichtigkeit sind, wurde speziell für die Übertragung geladener Moleküle, im Besonderen Nukleinsäuren der Versuch unternommen, die stark negative Ladung durch Verwendung DNA-neutralisierender Agenzien zu reduzieren. Neutralisierung ist mittels unterschiedlicher Moleküle möglich. Bindung von Proteinen, Proteinfragmenten oder Peptiden mit hoher Anzahl basischer Aminosäuren (Wienhues et al., DNA, 1987, 6:81-89, A novel method for transfection and expression of reconstituted DNAprotein complexes in eukaryotic cells) wurden ebenso zu diesem Zweck eingesetzt wie positiv geladene Polymere (Boussif et al. PNAS, 1995, 92:7297-7301, A versatile vector for gene and oligonucleotide transfer into cells in culture and in vivo: polyethylenimine) oder auch zweiwertige Ionen (Haberland et al. Biochim, Biophys Acta, 1999, 14:21-30, Calcium ions as efficient cofactor of polycation-mediated gene transfer). Für Komplexe aus DNA und Proteinen/Peptiden wie beispielsweise Polylysin, Protamin oder auch adenoviralen Zentralproteinen konnte gezeigt werden, dass diese eine direkte Verbesserung der Aufnahmeeffizienz von DNA in lebende Zellen auch ohne zusätzlichen Einsatz liposomaler Systeme bewirken (Wienhues et al. siehe oben). Hierbei bilden DNA und Proteine/Peptide homogene, dichtgepackte Nanopartikel (Polyplexe, auch Nanoplexe) aus, die hierdurch von enzymatischem Abbau besser geschützt sind. Diese Nanopartikel können weiterhin mittels endosomaler Transfektion sowie Elektroporation in Form von Lipopolyplexen in Zellen eingebracht werden, um dort die Transfektionseffizienz zu erhöhen (Chen et al. Biomaterials, 2011, 32:1412-1418, Transfection efficiency and intracellular fate of polycation liposomes combined with protamine). Die Erhöhung der Transfektionseffizienz wird hierbei vermutlich sowohl durch einen Protamin-vermittelten, verbesserten Austritt aus Lysosomen als auch verbesserten Transport in den Zellkern hervorgerufen.

Ähnliche Ergebnisse wie für kationische Proteine/Peptide sind auch für polykationische Polymere, wie beispielsweise Polyethylenimine (PEI) beschrieben (Boussif et al. PNAS, 1995, 92:7297-7301, A versatile vector for gene and oligonucleotide transfer into cells in culture and in vivo: polyethylenimine). Auch hier können somit Polyplexe in Anwesenheit von Nukleinsäuren aufgebaut und entweder direkt oder in Kombination mit Liposomen in Zellen eingeschleust werden, um dort mittels reduziertem enzymatischem Abbau der DNA, verbesserter Freisetzung aus Lysosomen sowie besserem Transport der DNA in den Zellkern die funktionelle Einbringung von Nukleinsäuren zu erhöhen.

Zweiwertige Ionen dienen typischerweise bei der Übertragung geladener Moleküle als unterstützende Einheit. In Kombination speziell mit endozytotischen Aufnahmemechanismen bauen zweiwertige Ionen, im Besonderen Ca²⁺, vermutlich Mikropräzipitate auf (Calziumphosphat für Ca²⁺). Derartige Mikropräzipitate besitzen membranolytische Aktivität und erwirken hierdurch vermutlich eine verbesserte Freisetzung der Nukleinsäuren aus Lysosomen, indem sie diese zerstören (Haberland et al. Biochim, Biophys Acta, 1999, 14:21-30, Calcium ions as efficient cofactor of polycation-mediated gene transfer).

Nachteilig für die Übertragung von Nukleinsäuren mittels endosomaler Aufnahme (Lipofektion) ist der natürlicherweise in Endosomen ablaufende Degradationsmechanismus der Nukleinsäuren innerhalb der Zellen. Mittels künstlicher Mechanismen müssen daher Endosomen an ihrer Funktionsweise gehindert und dazu gebracht werden sich aufzulösen und aufgenommene Stoffe freizugeben. Dieser Eingriff in den endosomalen Funktionsmechanismus stellt einen signifikanten Eingriff und damit Stress für lebende Zellen dar, wodurch natürliche Funktionsweisen verändert sein können. Das Stresslevel wird des Weiteren dadurch erhöht, dass die zur Übertragung von Nukleinsäuren aufgebauten Lipoplexe einen sehr hohen Anteil positiv geladener Lipide aufweisen, die von Zellen selbst kaum aufgebaut werden und daher zur Letalität führen können, aber selbst bei reduzierten Konzentrationen die Mikrodomänenstruktur biologischer Membranen signifikant schädigen bzw. verändern (Dass, J. Pharm Pharmacol, 2002, 54:593-601, Cytotoxicity issues pertinent to lipoplex-mediated gene therapy in-vivo). Hinzu kommt, dass unterschiedliche Zelltypen durch eine stark variierende Endozytoseaktivität gekennzeichnet sind, wodurch die Effizienz der Lipofektion stark beeinträchtigt sein kann.

Im Falle der Elektroporation sind die durch die Methodik hervorgerufenen Stresslevel derart hoch, dass bis zu 90% der eingesetzten Zellen die Prozedur nicht überleben (Vernhes et al. Bioelectrochem Bioenerg. 1999, 48:17-25, Chinese hamster ovary cells sensitivity to localized electrical stresses). Dieser Nachteil kommt ursächlich durch die Generierung unterschiedlich großer Nanoporen in der äußeren Zellmembran zustande, durch die der natürliche und genauestens regulierte lonenhaushalt der Zelle empfindlich gestört wird. Derartige Stressniveaus bewirken sowohl für im Fall der Elektroporation als auch Lipofektion inhomogene Übertragungsmengen an Lipoplexen wodurch auch nachfolgend die Analyse der zu untersuchenden Funktion einer erhöhten Heterogenität unterworfen ist.

Während hohe Stresswerte bei Zielzellen und niedrige Effizienzen bei der liposomalen Fusion deutlich reduziert sind, hat diese Methodik den Nachteil, sehr empfindlich auf Veränderungen der elektrostatischen Wechselwirkungen zwischen fusogenem Liposom sowie Zielmembran zu reagieren. Bereits geringe Mengen geladener Moleküle verändern hierbei die Wechselwirkung zwischen Liposomen und Zielmembran derart, dass nach Ankopplung der Liposomen eine nachfolgende Fusion beider Membranen teilweise bis vollkommen gehemmt wird und dadurch auch fusogene Liposomen nur noch endozytotisch mit den damit verbundenen Nachteilen aufgenommen werden. Da Mischungen aus kationischen Lipiden mit Polyanionen wie z.B. DNA oder RNA wie oben beschrieben zur Komplexierung in Lipoplexe neigen, ist bei Fusionshemmung ebenfalls eine derartige Komplexbildung mit hoher Übertragung positiv geladener Lipide wie mittels Elektroporation bzw. Lipofektion wahrscheinlich.

Aufgabe der Erfindung ist es daher, eine verbesserte Fusionsmischung zur Übertragung eines geladenen Moleküls bereitzustellen. Diese Aufgabe wird durch den Gegenstand von Anspruch 1 gelöst.

Erfindungsgemäß wird ein Verfahren zum Herstellen einer Fusionsmischung für eine Übertragung eines geladenen Moleküls in und/oder durch eine Lipidmembran bereitgestellt, mit den Schritten:
a) Bereitstellen einer Ausgangsmischung umfassend ein positiv geladenes amphipathisches Molekül A, ein aromatisches Molekül B mit hydrophobem Bereich und ein neutrales, amphipathisches Molekül C, wobei die Molekülsorten in einem Verhältnis A:B:C von 1 - 2 : 0,02 - 1 : 0 - 1 mol/mol vorliegen,
b) Erzeugen eines fusogenen Liposoms durch Aufnahme der Ausgangsmischung in einem wässrigen Lösungsmittel,
c) Bereitstellen eines geladenen Moleküls,
d) Ausbilden eines Komplexes aus dem geladenen Molekül und einem neutralisierenden Agens,
e) Inkubieren des Komplexes mit dem fusogenen Liposom, so dass eine Fusionsmischung erhalten wird

Es hat sich überraschenderweise herausgestellt, dass dieses Verfahren zu einer Mischung führt, die in zuverlässiger Weise eine Übertragung mittels Fusion in und/oder durch eine Zielmembran in Form einer Lipidmembran ermöglicht, bei der die übertragenen Moleküle ihre Funktion beibehalten und der gesamte Prozess mit niedrigen Stresswerten für die Zielmembran einhergeht. Diese Abfolge von Schritten (in der anspruchsgemäßen Reihenfolge) führt zu einer vorteilhaften Einbettung des Komplexes in das fusogene Liposom. Das entstehende fusogene Komplex-Liposom ist kein klassisches Lipoplex, das mittels Endozytose aufgenommen würde.

Überraschenderweise führt speziell die anspruchsgemäße Abfolge von Schritten zu den gewünschten hervorragenden Fusionseigenschaften. Ausgehend vom Stand der Technik bezüglich des Transportes von elektrisch neutralen Molekülen z.B. in Zellen, würde der Fachmann eigentlich davon ausgehen, dass es am effektivsten wäre, wenn man das Produkt aus Schritt e) für die in Schritt b) verwendete wässrige Lösung hernehmen würde. Das würde bedeuten, dass man erst aus geladenem Molekül und neutralisierendem Agens eine Lösung herstellt, mit der man dann die Ausgangsmischung mischt (und quellen lässt), um die fusogenen Liposome herzustellen. Diese Vorgehensweise ist deswegen naheliegender, da man erwartet, dass der Komplex aus geladenem Molekül und neutralisierendem Agens mit einer höheren Wahrscheinlichkeit in das Lumen des neugeformten Liposoms aufgenommen wird, wenn der Komplex in der Lösung vorhanden ist, die zum Quellen verwendet wird. Der Partikeltransport funktioniert jedoch überraschenderweise wesentlich effektiver, wenn die Komplexe erst zu den Liposomen gegeben werden, nachdem diese geformt wurden.

Bei der Zielmembran/Lipidmembran kann es sich um eine Biomembran (oder einen Bestandteil davon) oder eine künstlich erzeugte Lipidmembran handeln. Beispiele einer Lipidmembran sind eine Zellmembran, einschließlich Plasma- oder Zellorganellmembranen, ein Bestandteil einer Zellmembran, eine aufgereinigte Biomembran oder auch ein künstliches Membransystem.

Das geladene Molekül kann dissoziert (also beispielsweise protoniert) oder in ionischer Form vorliegen. Bei dem geladenen Molekül kann es sich insbesondere um ein Protein, ein Peptid, eine Aminosäure, ein Polynukleotid, eine Nukleinsäure, ein Poly-, Oligo- oder Disaccharid, ein Antibiotikum, ein Antiseptikum, ein Zytostatikum, ein Immunsuppressivum, ein therapeutisch relevantes Polymer (z.B. Polymaleinsäure, Hyaluronsäure, funtionalisiertes Dextran, Hydrogele), ein Dendrimer, ein Chelator, ein oberflächenfunktionalisiertes Nano- oder Mikropartikel (z.B. Polystyren, Ferritin), ein Mikroschwimmer und/oder ein Farbstoff handeln. Das geladene Molekül kann natürlichen Ursprungs sein, wie z. B. aus Zellen extrahierte und ggf. aufgereinigte Proteine oder Polynukleotide, oder ein synthetisch hergestelltes Molekül sein, wie z.B. mehrfach geladene Polypeptide oder Polynukleotide.

In Schritt a) können die Moleküle der Ausgangsmischung bereits in einer wässrigen Lösung vorliegen oder als trockenes Lipidgemisch bzw. aus einem organischen Lösungsmittel heraus nach Trocknung in wässrige Lösung überführt werden. Die wässrige Lösung stellt damit einen Liposomenpuffer dar. Dieser Puffer weist vorzugsweise eine Osmolarität von weniger als 100 mOsm auf. Sein pH-Wert kann in einem Bereich von 7,0 bis 8,0 liegen. Die wässrige Lösung kann beispielsweise HEPES, TRIS, HEPPS oder auch Phosphatpuffer sein.

Schritt d) kann derart erfolgen, dass der Komplex ein Zeta-Potential von -50 mV bis 0 mV, insbesondere von -50 mV bis -10 mV, aufweist. Dies führt zu einer verbesserten Zuverlässigkeit der Übertragung geladener Moleküle mittels Fusion und reduziert deutlich die Wahrscheinlichkeit einer Aufnahme mittels Endozytose. Im Folgenden wird der Komplex aus geladenem Molekül und neutralisierendem Agens manchmal auch Komplex A genannt.

Das neutralisierende Agens weist insbesondere eine positive oder negative Ladung auf. Die Ladung des neutralisierenden Agens weist ein zur Ladung des geladenen Moleküls entgegengesetztes Vorzeichen auf. Im Falle von negativ geladenen Molekülen (beispielsweise DNA, RNA oder siRNA) wird damit ein positiv geladenes Agens, im Falle von positiv geladenen Molekülen (beispielsweise basische Peptide/Proteine (Histone, Avidin) oder kationische Polymere) ein negativ geladenes Agens verwendet.

Das neutralisierende Agens für ein negativ geladenes Molekül führt dazu, dass die Gesamtladung des Komplexes im Vergleich zur Ladung des geladenen Moleküls in Richtung einer neutralen Ladung verschoben wird. Das neutralisierende Agens für ein positiv geladenes Molekül führt dazu, dass die Gesamtladung des Komplexes im Vergleich zur Ladung des geladenen Moleküls über eine neutrale Ladung hinaus in den anionischen Bereich verschoben wird. Unabhängig von dem Vorzeichen der Ladung des geladenen Moleküls hat sich der angegebene Bereich des Zeta-Potenzials als besonders vorteilhaft erwiesen.

Im Falle negativ geladener Moleküle kann für die Neutralisierung/Ladungsverschiebung als neutralisierendes Agens insbesondere ein polykationisches Polymer oder ein basisches (und damit positiv geladenes) Peptid oder Protein eingesetzt werden. Beispielsweise kann das neutralisierende Agens Polyethylenimin (PEI) (beliebiger Kettenlänge), Poly-L-Lysin, Protamin, Chitosan, eine Histon-Untereinheit oder ein adenovirales Kernprotein sein. Im Falle positiv geladener Moleküle, wie beispielsweise Streptavidin, kann als neutralisierendes Agens insbesondere ein polyanionisches Molekül, beispielsweise Hyaluronsäure, Dextrane oder langkettige Fettsäuren verwendet werden.

Das Mischungsverhältnis von geladenem, insbesondere negativ geladenem, Molekül und neutralisierendem Agens kann 1:0,05-1,2 (g/g), insbesondere 1:0,8-1 (g/g), betragen. Dies führt zu einem vorteilhaften Neutralisieren der Oberflächenladung.

Schritt d) kann ein Inkubieren des geladenen Moleküls mit dem neutralisierenden Agens umfassen. Das Inkubieren kann beispielsweise während eines Zeitraums von 30 Sekunden bis 120 Minuten, insbesondere während eines Zeitraums von 1 Minute bis 60 Minuten erfolgen.

Bei den zuvor beschriebenen Verfahren kann vor Schritt e) ein Hinzufügen von Kationen erfolgen, um den Komplex zu stabilisieren. Eine solche Stabilisierung des Komplexes A erhöht die Übertragungseffizienz. Das Hinzufügen von Kationen kann während und/oder nach Durchführung des Schritts d) erfolgen.

Bei den Kationen kann es sich um einwertige, zweiwertige oder dreiwertige Kationen handeln. Beispiele für mögliche Kationen sind Na⁺, K⁺, Ca²⁺, Mg²⁺ und Fe³⁺.

Die Kationen können insbesondere mit einer Konzentration von 0 bis 1 mM hinzugefügt werden.

Bei den zuvor beschriebenen Verfahren kann Schritt d) ein Hinzufügen von Albumin umfassen.

Es wurde überraschenderweise festgestellt, dass die Zugabe von Albumin die Übertragungseigenschaft von geladenen Molekülen deutlich erhöhen kann. Dies ist insbesondere deshalb erstaunlich, da bei bereits bekannten Systemen mit fusogenen Liposomen die Anwesenheit von Proteinen, beispielsweise Albumin, einen deutlich negativen Einfluss auf die Fusogenität und damit Übertragungseffizienz hat. Bei dem erfindungsgemäßen Verfahren hingegen stellt man bei der Hinzufügung von Albumin eine weitere Verbesserung der Fusogenität fest. Das Albumin kann in einer Konzentration von 10 µM bis 5 mM, insbesondere von 10 bis 500 µM, hinzugegeben werden.

Bei den zuvor beschriebenen Verfahren kann vor, während und/oder nach Schritt e) ein Lipidmembran-destabilisierendes Agens hinzugefügt werden. Das Hinzufügen kann nach dem Schritt d) erfolgen. Es kann also beispielsweise dem fusogenen Liposom vor dem Inkubieren mit dem Komplex A hinzugegeben werden. Das Hinzufügen kann auch während des Inkubierens oder danach erfolgen. Damit wird eine anschließend stattfindende Fusion erleichtert.

Das Lipidmembran-destabilisierende Agens kann ein Detergens sein. Es kann insbesondere ein Kopf-zu-Ketten-Querschnittsverhältnis von mehr als 1:1, insbesondere von wenigstens 2:1, aufweisen. Bei derartigen Kopf-zu-Ketten-Querschnittsverhältnissen erhöht sich vorteilhaft die destabilisierende Wirkung des Detergens.

Das Lipidmembran-destabilisierende Agens kann neutral geladen sein. Beispiele eines Lipidmembran-destabilisierende Agens sind Triton X-100 (C₁₄H₂₂O(C₂H₄O)*ₙ*), Tween 20 (Polysorbat 20) und Octylglucopyramosid.

Die Konzentration des destabilisierenden Agens kann vorzugsweise unter seiner kritischen Mizellierungskonzentration (CMC) liegen. Dies reduziert die Gefahr, dass die fusogenen Liposome in ihrer Struktur aufgelöst werden.

Bei den zuvor beschriebenen Verfahren können Schritt b) und/oder Schritt e) ein Durchführen einer Ultraschallbehandlung und/oder ein Durchführen einer Hochdruck-Homogenisierung umfassen. In Schritt b) unterstützt die Ultraschallbehandlung oder die Hochdruck-Homogenisierung die Generierung einer homogenen Suspension. In Schritt e) wird die Einbettung des Komplexes A in das fusogene Liposom durch die Ultraschallbehandlung oder die Hochdruck-Homogenisierung signifikant verbessert.

Die Ultraschallbehandlung kann bei einer Frequenz von 20 bis 70 kHz, insbesondere von 25 bis 50 kHz durchgeführt werden. Sie kann während eines Zeitraums von 1 bis 60 Minuten, insbesondere 5 bis 30 Minuten, erfolgen. Die Leistung kann bei 50 bis 1200 W, insbesondere 50 bis 1000 W, liegen.

Bei den beschriebenen Verfahren kann nach Schritt e) ein Verdünnen mit einem Puffer mit einer Osmolarität von 200 mOsm oder höher erfolgen. Grundsätzlich kann der Puffer ein wässriger Puffer sein. Der Puffer kann ein Zellkulturmedium (mit oder ohne Zusätze, bspw. Serum, Antibiotikum) sein.

Der Puffer kann einen pH-Wert von 5 bis 10, insbesondere von 7 bis 9, aufweisen. Dies führt zu verbesserten Fusogenitäten und Übertragungseffizienzen. Beispielhafte Puffer sind PBS (phosphate buffered saline), TBS (Tris buffered saline), MOPS (3-(N-Morpholino)propansulfonsäure-Puffer, Carbonat-Puffer oder HBSS (Hank's balanced salt solution) mit einer Osmolarität von 300 mOsm +/- 10%.

Das Verdünnen kann in einem Bereich von 1:10 bis 1:250 (Komplex A-Liposomen : Puffer), insbesondere 1:10 bis 1:200, erfolgen.

Während des Verdünnens und/oder nach dem Verdünnen können eine Ultraschallbehandlung und/oder eine Druckbeaufschlagung durchgeführt werden. Es hat sich herausgestellt, dass eine derartige Ultraschallbehandlung und/oder Druckbeaufschlagung eine Aggregation des Systems der fusogenen Komplex A-Liposomen vermeidet und die Anzahl aktiver Liposomen erhöht. Dadurch lässt sich die Effizienz und Homogenität der Übertragung verbessern. Für die Ultraschallbehandlung können die Verfahrensparameter der bereits oben genannten Ultraschallbehandlung einzeln oder in Kombination verwendet werden.

Bei den zuvor beschriebenen Verfahren können das Molekül A und/oder das Molekül C ein Lipid oder ein Lipidanalogon sein. Ein Lipidanalogon ist ein Lipid, das nicht aus der Natur heraus gebildet werden kann; es wird künstlich erzeugt. Lipide und Lipidanaloga haben sich als besonders vorteilhaft für die Durchführung von Fusion erwiesen.

Das Molekül A kann in seinem hydrophoben Bereich einen C₁₀-C₃₀―Anteil haben. Dieser kann Doppelbindungen aufweisen. Solche Doppelbindungen können die Elastizität der Membran des entstehenden Liposoms erhöhen, was zu einer Erleichterung der Fusion des Liposoms mit der Zellmembran führt. Geeignete Beispiele für Molekül A sind
- A1:: 1,2-Dioleoyl-3-Trimethylammonium-Propan Clorid (DOTAP)
- A2:: N-(2,3-Dioleyloxypropyl)-N, N, N-Trimetylammonium Chlorid (DOTMA)
- A3: Dimetyl-Dioctadecyl Ammonium Bromid (DDAB)
- A4:: (1-[2-(Oleoyloxy)Ethyl]-2-Oleyl-3-(2-Hydroxyethyl)Imidazolinium Chlorid (DOTIM).
- A5:: 3ß-[N-(N',N'-dimethylaminoethane)-carbamoyl]cholesterol hydrochloride (DC-Cholesterol)
- A6:: 1,2-dilauroyl-sn-glycero-3-ethylphosphocholine (chloride salt) (EPC)
- A7:: N1-[2-((1S)-1-[(3-aminopropyl)amino]-4-[di(3-amino-propyl)amino]butylcarboxamido)ethyl]-3,4-di[oleyloxy]-benzamide (MVL5)

Der Anteil des Moleküls A in der Ausgangsmischung kann bis zu 95 wt/wt% betragen. Besonders vorteilhaft sind Moleküle mit den oben genannten Eigenschaften und einem kritischen Packungsparameter (CPP, siehe unten) unterhalb von 1,5.

Das Molekül C kann in seinem hydrophoben Bereich einen C₁₀-C₃₀―Anteil haben. Dieser kann Doppelbindungen aufweisen. Vorzugsweise haben sowohl der hydrophile Bereich als auch der hydrophobe Bereich einen neutralen Charakter. Dies führt zu einer vorteilhaften Neutralisierung der Ladungsdichte und der abstoßenden Kräfte zwischen positiv geladenen Molekülen der Molekülsorte A. Auf diese Weise wird eine Stabilisierung des Systems erreicht. Der Anteil des Moleküls C in der Ausgangsmischung kann bis zu 60 wt/wt% betragen. Geeignete Beispiele für Molekül C sind beispielsweise:
- C1:: Phosphatidylethanolamine (bspw. 1,2-Dioleoyl-sn-Glycero-3-Phosphoethanolamine, 1,2-Dipalmitoyl-sn-Glycero-3-Phosphoethanolamine, 1,2-Dimiristoyl-sn-Glycero-3-Phosphoethanolamine, 1,2-Dielaidoylsn-Glycero-3-Phosphoethanolamine, 1,2-Diphytanolsn-Glycero-3-Phosphoethanolamine, 1,2-Dilinoleoyl*sn*-Glycero-3-Phospho-ethanolamine),
- C2:: Ceramide (bspw. C18 Ceramide (d18:1/18:0) N-stearoyl-D-*erythro*-sphingosine)
- C3:: Cholesterol

Vorteilhaft für die Verwendung als Molekülsorte C sind Phospholipide, im besonderen Phosphoethanolamine, Glycolipide, im Besonderen Ceramide sowie Sterole und Cholesterol.

Das Molekül A und/oder das Molekül C können bei Kontakt mit Wasser einen planaren oder nahezu planaren Bilayer ausbilden. Damit wird die Generierung von Liposomen in vorteilhafter Weise ermöglicht.

Ein planarer Bilayer wird ausgebildet, wenn der kritische Packungsparameter CPP 1 ist. Ein nahezu planarer Bilayer liegt vor, wenn der kritische Packungsparameter zwischen 0,5 und 1 oder zwischen 1 und 1,5 liegt. Insbesondere kann der kritische Packungsparameter zwischen 1 und 1,5 liegen.

Der kritische Packungsparameter ist definiert als *CPP* = *ν*/*al,* wobei v das Volumen des hydrophoben/lipophilen Kettenbereichs, *a* die Querschnittsfläche des hydrophilen Kopfbereichs und *l* die Länge der lipophilen Kette sind (siehe M. Salim et al., Amphiphilic designer nanocarriers for controlled release: from drug delivery to diagnostics, Med. Chem. Commun., 2014, 5, 1602, Abschnitt 4a).

Das aromatische Molekül B kann selbst eine aromatische Verbindung darstellen oder mindestens eine aromatische Gruppe enthalten. Das Molekül B weist somit ein zyklisches Strukturmotiv (aromatischer Ring) aus konjugierten Doppelbindungen und/oder freien Elektronenpaaren oder unbesetzten p-Orbitalen auf, das der Hückel-Regel genügt. Der aromatische Ring kann einen Elektronegativitätsunterschied Δ*χ* zwischen kovalent verbundenen Nachbaratomen von mindestens 0,4, vorzugsweise von 0,5 bis 2, aufweisen. Dies erhöht die Polarisierbarkeit und führt zu einer weiter verbesserten Fusion. Der Anteil des Moleküls B in der Ausgangsmischung kann bis zu 40 wt/wt% betragen.

### Geeignet Beispiele für Molekül B sind

Fluoreszenzfarbstoffe oder farbstoffmarkierte Lipide, z. B.:
- B1:: DiOC₁₈(3)3,3'-Dioctadecyloxacarbocyanine Perchlorate (DiO),
- B2:: 1,1'-Dioctadecyl-3,3,3',3'-Tetramethylindotricarbocyanine lodide (DiR),
- B3:: N-(4,4-Difluoro-5,7-Dimethyl-4-Bora-3a,4a-Diaza-s-Indacene-3-Propionyl)-1,2-Dihexadecanoyl-*sn*-Glycero-3-Phosphoethanolamine, Triethylammonium Salz (BO-DIPY FL DHPE),
- B4 :: 2-(4,4-Difluoro-5-Methyl-4-Bora-3a,4a-Diaza-*s*-Indacene-3-Dodecanoyl)-1-Hexadecanoyl-sn-Glycero-3-Phosphocholine (β-BODIPY 500/510 C₁₂-HPC),
- B5 :: Lissamin Rhodamine B 1,2-Diolenoyl-*sn*-Glycero-3-Phosphoethanolamine, Triethy-lammonium Salz (LR-DOPE),
- B6 :: Lissamin Rhodamine B 1,2-Dihexadecanoyl-*sn*-Glycero-3-Phosphoethanolamine, Triethylammonium Salz, (LR-DHPE),
- B7:: Texas Red 1,2-Dihexadecanoyl-*sn*-Glycero-3-Phosphoethanolamine, Triethylammonium Salz (Texas Red DHPE),
- B8:: *N*-(7-nitrobenz-2-oxa-1,3-diazol-4-yl)-1,2-Diolenoyl-*sn*-Glycero-3-Phosphoethanolamine, Triethylammonium Salz (NBD-DOPE),
- B9:: 1,2-Dioleoyl-*sn*-Glycero-3-Phosphoethanolamine-N-(Carboxyfluorescein), Ammonium Salz, (Fluorescein-DOPE),
- B10:: 1,2-Dioleoyl-sn-Glycero-3-Phosphoethanolamine-N-(1-pyrenesulfonyl), Ammonium Salz, (Pyrene-DOPE),
- B11:: 1-Hexadecanoyl-2-(1-Pyrenedecanoyl)-*sn*-Glycero-3-Phosphocholine (β-Pyrene C₁₀-HPC),
- B12:: Polyphenole (bspw. Resveratrol, Curcumin, 5-Hydroxyflavon),
- B13:: Vitamine (bspw. Vitamin-E, Vitamin-A oder Vitamin-K),
- B14:: Zytostatika (bspw. Doxorubicin, Paclitaxel).

Andere pharmakologisch wirksame, aromatische Substanzen sind ebenfalls möglich.

Durch Wahl eines fluoreszierenden Moleküls B zur Herstellung fusogener Liposomen kann dieses nach Fusion in der Zielmembran leicht nachgewiesen werden. Der Nachweis ist über klassische Systeme wie beispielsweise Fluoreszenzmikroskopie oder Durchflusszytometrie möglich. Hierdurch kann ohne Zeitverlust die für den effizienten Transfer geladener Moleküle notwendige Fusion direkt verifiziert werden.

Alle Moleküle, welche die Kriterien für die Molekülsorte A erfüllen, können in beliebiger Weise mit allen Molekülen, welche die Kriterien für die Molekülsorte B erfüllen, und mit allen Molekülen, welche die Kriterien für die Molekülsorte C erfüllen, kombiniert werden, sofern das erfindungsgemäße Mischungsverhältnis vorliegt.

Insbesondere sind alle in der folgenden Tabelle aufgeführten Kombinationen von Molekülen A/B/C als Ausgangsmischung möglich, wobei obige Abkürzungen A1, ..., A7, B1, ..., B14, C1, C2, C3 für die verschiedenen, beispielhaft aufgeführten Moleküle verwendet werden:

| | | | | | | |
|---|---|---|---|---|---|---|
| A1/B1/C1 | A2/B1/C1 | A3/B1/C1 | A4/B1/C1 | A5/B1/C1 | A6/B1/C1 | A7/B1/C1 |
| A1/B1/C2 | A2/B1/C2 | A3/B1/C2 | A4/B1/C2 | A5/B1/C2 | A6/B1/C2 | A7/B1/C2 |
| A1/B1/C3 | A2/B1/C3 | A3/B1/C3 | A4/B1/C3 | A5/B1/C3 | A6/B1/C3 | A7/B1/C3 |
| A1/B2/C1 | A2/B2/C1 | A3/B2/C1 | A4/B2/C1 | A5/B2/C1 | A6/B2/C1 | A7/B2/C1 |
| A1/B2/C2 | A2/B2/C2 | A3/B2/C2 | A4/B2/C2 | A5/B2/C2 | A6/B2/C2 | A7/B2/C2 |
| A1/B2/C3 | A2/B2/C3 | A3/B2/C3 | A4/B2/C3 | A5/B2/C3 | A6/B2/C3 | A7/B2/C3 |
| A1/B3/C1 | A2/B3/C1 | A3/B3/C1 | A4/B3/C1 | A5/B3/C1 | A6/B3/C1 | A7/B3/C1 |
| A1/B3/C2 | A2/B3/C2 | A3/B3/C2 | A4/B3/C2 | A5/B3/C2 | A6/B3/C2 | A7/B3/C2 |
| A1/B3/C3 | A2/B3/C3 | A3/B3/C3 | A4/B3/C3 | A5/B3/C3 | A6/B3/C3 | A7/B3/C3 |
| A1/B4/C1 | A2/B4/C1 | A3/B4/C1 | A4/B4/C1 | A5/B4/C1 | A6/B4/C1 | A7/B4/C1 |
| A1/B4/C2 | A2/B4/C2 | A3/B4/C2 | A4/B4/C2 | A5/B4/C2 | A6/B4/C2 | A7/B4/C2 |
| A1/B4/C3 | A2/B4/C3 | A3/B4/C3 | A4/B4/C3 | A5/B4/C3 | A6/B4/C3 | A7/B4/C3 |
| A1/B5/C1 | A2/B5/C1 | A3/B5/C1 | A4/B5/C1 | A5/B5/C1 | A6/B5/C1 | A7/B5/C1 |
| A1/B5/C2 | A2/B5/C2 | A3/B5/C2 | A4/B5/C2 | A5/B5/C2 | A6/B5/C2 | A7/B5/C2 |
| A1/B5/C3 | A2/B5/C3 | A3/B5/C3 | A4/B5/C3 | A5/B5/C3 | A6/B5/C3 | A7/B5/C3 |
| A1/B6/C1 | A2/B6/C1 | A3/B6/C1 | A4/B6/C1 | A5/B6/C1 | A6/B6/C1 | A7/B6/C1 |
| A1/B6/C2 | A2/B6/C2 | A3/B6/C2 | A4/B6/C2 | A5/B6/C2 | A6/B6/C2 | A7/B6/C2 |
| A1/B6/C3 | A2/B6/C3 | A3/B6/C3 | A4/B6/C3 | A5/B6/C3 | A6/B6/C3 | A7/B6/C3 |
| A1/B7/C1 | A2/B7/C1 | A3/B7/C1 | A4/B7/C1 | A5/B7/C1 | A6/B7/C1 | A7/B7/C1 |
| A1/B7/C2 | A2/B7/C2 | A3/B7/C2 | A4/B7/C2 | A5/B7/C2 | A6/B7/C2 | A7/B7/C2 |
| A1/B7/C3 | A2/B7/C3 | A3/B7/C3 | A4/B7/C3 | A5/B7/C3 | A6/B7/C3 | A7/B7/C3 |
| A1/B8/C1 | A2/B8/C1 | A3/B8/C1 | A4/B8/C1 | A5/B8/C1 | A6/B8/C1 | A7/B8/C1 |
| A1/B8/C2 | A2/B8/C2 | A3/B8/C2 | A4/B8/C2 | A5/B8/C2 | A6/B8/C2 | A7/B8/C2 |
| A1/B8/C3 | A2/B8/C3 | A3/B8/C3 | A4/B8/C3 | A5/B8/C3 | A6/B8/C3 | A7/B8/C3 |
| A1/B9/C1 | A2/B9/C1 | A3/B9/C1 | A4/B9/C1 | A5/B9/C1 | A6/B9/C1 | A7/B9/C1 |
| A1/B9/C2 | A2/B9/C2 | A3/B9/C2 | A4/B9/C2 | A5/B9/C2 | A6/B9/C2 | A7/B9/C2 |
| A1/B9/C3 | A2/B9/C3 | A3/B9/C3 | A4/B9/C3 | A5/B9/C3 | A6/B9/C3 | A7/B9/C3 |
| A1/B10/C1 | A2/B10/C1 | A3/B10/C1 | A4/B10/C1 | A5/B10/C1 | A6/B10/C1 | A7/B10/C1 |
| A1/B10/C2 | A2/B10/C2 | A3/B10/C2 | A4/B10/C2 | A5/B10/C2 | A6/B10/C2 | A7/B10/C2 |
| A1/B10/C3 | A2/B10/C3 | A3/B10/C3 | A4/B10/C3 | A5/B10/C3 | A6/B10/C3 | A7/B10/C3 |
| A1/B11/C1 | A2/B11/C1 | A3/B11/C1 | A4/B11/C1 | A5/B11/C1 | A6/B11/C1 | A7/B11/C1 |
| A1/B11/C2 | A2/B11/C2 | A3/B11/C2 | A4/B11/C2 | A5/B11/C2 | A6/B11/C2 | A7/B11/C2 |
| A1/B11/C3 | A2/B11/C3 | A3/B11/C3 | A4/B11/C3 | A5/B11/C3 | A6/B11/C3 | A7/B11/C3 |
| A1/B12/C1 | A2/B12/C1 | A3/B12/C1 | A4/B12/C1 | A5/B12/C1 | A6/B12/C1 | A7/B12/C1 |
| A1/B12/C2 | A2/B12/C2 | A3/B12/C2 | A4/B12/C2 | A5/B12/C2 | A6/B12/C2 | A7/B12/C2 |
| A1/B12/C3 | A2/B12/C3 | A3/B12/C3 | A4/B12/C3 | A5/B12/C3 | A6/B12/C3 | A7/B12/C3 |
| A1/B13/C1 | A2/B13/C1 | A3/B13/C1 | A4/B13/C1 | A5/B13/C1 | A6/B13/C1 | A7/B13/C1 |
| A1/B13/C2 | A2/B13/C2 | A3/B13/C2 | A4/B13/C2 | A5/B13/C2 | A6/B13/C2 | A7/B13/C2 |
| A1/B13/C3 | A2/B13/C3 | A3/B13/C3 | A4/B13/C3 | A5/B13/C3 | A6/B13/C3 | A7/B13/C3 |
| A1/B14/C1 | A2/B14/C1 | A3/B14/C1 | A4/B14/C1 | A5/B14/C1 | A6/B14/C1 | A7/B14/C1 |
| A1/B14/C2 | A2/B14/C2 | A3/B14/C2 | A4/B14/C2 | A5/B14/C2 | A6/B14/C2 | A7/B14/C2 |
| A1/B14/C3 | A2/B14/C3 | A3/B14/C3 | A4/B14/C3 | A5/B14/C3 | A6/B14/C3 | A7/B14/C3 |

Dabei steht beispielsweise "A1/B1/C1" für die Mischung DOTAP/DiO/Phosphatidylethanolamine oder "A3/B4/C2" für die Mischung DDAB/β-BODIPY 500/510 C₁₂-HPC/ C18-Ceramide .

Die Erfindung stellt weiterhin eine Fusionsmischung erhältlich nach einem der zuvor beschriebenen Verfahren bereit.

Darüber hinaus stellt die Erfindung die Verwendung einer derartigen Fusionsmischung zur Fusion mit einer Lipidmembran bereit. Bei der Lipidmembran kann es sich um eine Biomembran (oder einen Bestandteil davon) oder eine künstlich erzeugte Lipidmembran handeln. Dabei kann die Lipidmembran insbesondere eine Zellmembran, einschließlich Plasma- oder Zellorganellmembranen, ein Bestandteil einer Zellmembran, eine aufgereinigte Biomembran oder auch ein künstliches Membransystem sein.

Die Erfindung stellt auch ein Verfahren zur Übertragung eines geladenen Moleküls in und/oder durch eine Lipidmembran mittels Fusion bereit, mit den Schritten:
Durchführen eines der oben beschriebenen Verfahren zum Herstellen einer Fusionsmischung,

In-Kontakt-Bringen der Fusionsmischung mit einer Lipidmembran, sodass die Fusionsmischung mit der Lipidmembran fusioniert.

Es hat sich überraschenderweise herausgestellt, dass mit den oben beschriebenen Verfahren hergestellte Komplex A-Liposome direkt nach Kontakt mit der Zielmembran fusionieren und den Komplex A in das durch die Zielmembran umgebende Lumen abgeben.

Bei dem Verfahren kann die Lipidmembran eine Biomembran (oder einen Bestandteil davon) oder eine künstlich erzeugte Lipidmembran sein. Die Lipidmembran kann eine Zellmembran, einschließlich Plasma- oder Zellorganellmembranen, ein Bestandteil einer Zellmembran, eine aufgereinigte Biomembran oder auch ein künstliches Membransystem sein.

Der Schritt des In-Kontakt-Bringens kann in Suspension und/oder mit einer auf einer Substratoberfläche adhärierten Lipidmembran durchgeführt werden. Der Schritt des In-Kontakt-Bringens kann für einen Zeitraum von 1 Minute bis 60 Minuten, insbesondere für 5 bis 30 Minuten, erfolgen. Bei diesem Schritt kann die Temperatur im Bereich von 20 bis 45°C liegen. Der pH-Wert kann im Bereich von 6 bis 9 liegen. Die Osmolarität kann Werte von mindestens 200 mOsm aufweisen.

Im Weiteren wird die Erfindung an Hand von Ausführungsbeispielen und der beigefügten Figuren näher erläutert. Dabei illustriert bzw. zeigt:
- Fig. 1a:: die Fusogenität klassischer Fusionssysteme bei ansteigender Konzentration geladener Moleküle (DNA);
- Fig. 1b:: die Fusogenität klassischer Fusionssysteme bei ansteigender Konzentration geladener Partikel;
- Fig. 2a:: den Einfluss der Neutralisierung geladener Moleküle (DNA) mittels Peptid auf die Fusogenität fusogener Liposomen sowie die Übertragungseffizienz;
- Fig. 2b:: den Einfluss der Neutralisierung geladener Moleküle (RNA) mittels Peptid auf die Fusogenität fusogener Liposomen sowie die Übertragungseffizienz;
- Fig. 3:: den Einfluss der Neutralisierung geladener Moleküle (RNA) mittels polyprotonierter Polymere auf Fusogenität und Übertragungseffizienz;
- Fig. 4:: die Zetapotentialverschiebung in Abhängigkeit der Komplexzusammensetzung;
- Fig. 5:: den Einfluss der Membran-Destabilisierung auf Fusogenität und Übertragung geladener Moleküle;
- Fig. 6:: den Einfluss zusätzlicher Kationen auf Fusogenität und Übertragung geladener Moleküle;
- Fig. 7:: den Einfluss des pH-Wertes des Liposomenpuffers auf Fusogenität und Übertragung geladener Moleküle;
- Fig. 8:: den Einfluss des pH-Wertes des Verdünnungspuffers (PBS) auf Fusogenität und Übertragung geladener Moleküle;
- Fig. 9:: die Abhängigkeit der Fusogenität und Übertragbarkeit geladener Moleküle von der Zusammensetzung der fusogenen Liposomen;
- Fig. 10:: die universelle Übertragbarkeit geladener Moleküle in unterschiedliche Zelllinien und Primärzellen mittels angepasster Fusion;
- Fig. 11:: den Einfluss zusätzlicher Ultraschallbehandlung auf Fusogenität und Übertragung geladener Moleküle;
- Fig. 12:: herkömmliche fusogene Liposomen und ein erfindungsgemäßes Fusionssystem im Vergleich;
- Fig. 13:: unterschiedliche Lipidzusammensetzung (Komponenten A, B und C) funktioneller fusogener Liposomen;
- Fig. 14a:: den Einfluss von Albuminen auf die Übertragung geladener Moleküle am Beispiel von DNA;
- Fig. 14b:: den Einfluss von Albuminen auf die Übertragung geladener Moleküle am Beispiel von RNA.

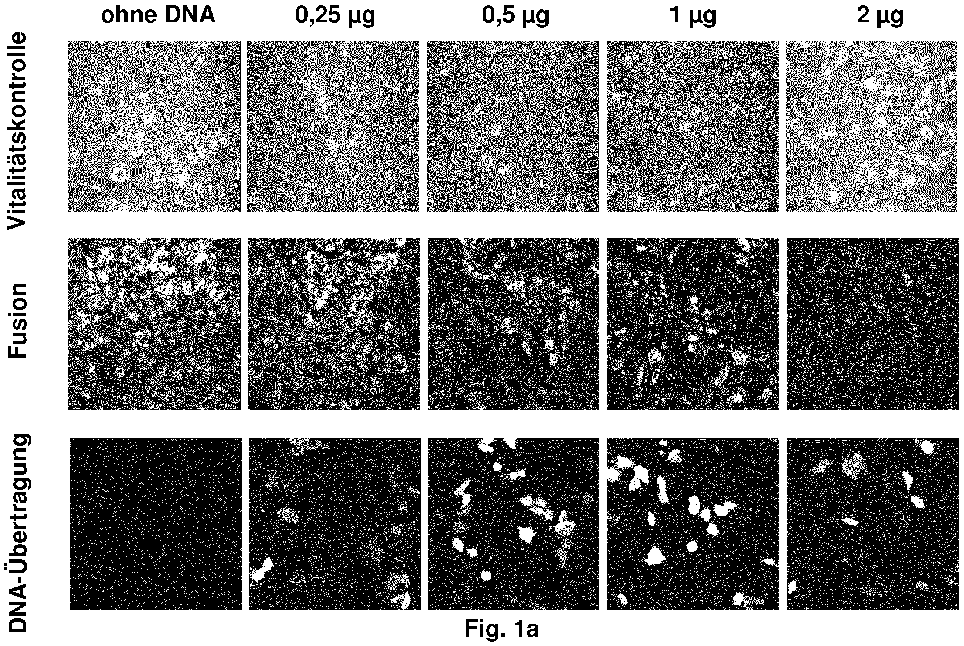

Fig. 1a illustriert die stark reduzierte Fusogenität klassischer Fusionssysteme (keine Neutralisierung geladener Moleküle, keine Pufferanpassungen, keine zusätzlichen Ionen, keine pH-Wertanpassungen, keine zusätzliche Ultraschallbehandlung) bei ansteigender Konzentration geladener Moleküle (DNA). Als DNA wurde beispielhaft auch in den nachfolgenden Beispielen ein Konstrukt verwendet, das nach funktioneller Einbringung von den Zellen in ein grün leuchtendes Protein (GFP) umgeschrieben (translatiert) wird und so leicht mittels Mikroskopie detektiert werden kann. Zur Überprüfung der Übertragungseffizienz geladener Moleküle durch klassische, fusogene Liposome der Zusammensetzung: positiv geladenens Lipid (DOTAP), fusogenes Molekül (DiR) und neutrales Lipid (DOPE) im Gewichtsverhältnis 1:0,1:1, wurden 10 µl einer 3 mM Lösung verwendet und mit steigenden Konzentrationen an DNA inkubiert. Die Inkubation erfolgte in 20 mM HEPES (2-(4-(2-Hydroxyethyl)-1-piperazinyl)-ethansulfonsäure) pH 7,4 für 10 min bei Raumtemperatur (RT).

Anschließend wurden die fusogenen Lipid-DNA-Liposome im Ultraschallbad bei 36 kHz und 200 W für 20 min bei RT behandelt, um fusogene Liposome mit einer mittleren Größe von etwa 340 nm zu erhalten. 10 µl der entstehenden fusogenen Lipid-DNA-Liposome wurden mit PBS (phosphatgepufferte Salzlösung) im Verhältnis 1:50 verdünnt und nochmals für 20 min unter gleichen Bedingungen wie zuvor im Ultraschallbad inkubiert, um eine möglichst hohe Anzahl fusogener Liposome zu erhalten. Die verdünnten Liposomen wurden anstelle des Zellkulturmediums zu adhärenten CHO (Chinese hamster ovary) Zellen gegeben, die zuvor in einer Dichte von 15.000 Zellen pro cm² ausgesät wurden, und für 20 min bei 37°C inkubiert.

Fusion wurde durch Austausch der Fusionslösung gegen Zellkulturmedium gestoppt. 24 Stunden nach Beendigung der Fusionsreaktion wurde mittels Fluoreszenzmikroskopie die Fusionseffizienz (Mitte) als auch die funktionelle Übertragung des Plasmids mittels Proteinexpressionsanalyse (unten) überprüft. Man erkennt die starke Reduktion der Fusogenität der Liposome mit steigender Konzentration an geladenem Molekül. Gleichzeitig erfolgt für keine einzige Konzentration eine funktionelle Übertragung der DNA (d.h. Expression der kodierenden Sequenz und damit Bildung des grün-fluoreszierenden Proteins (helles Signal)) mit einer Effizienz von mehr als 10%.

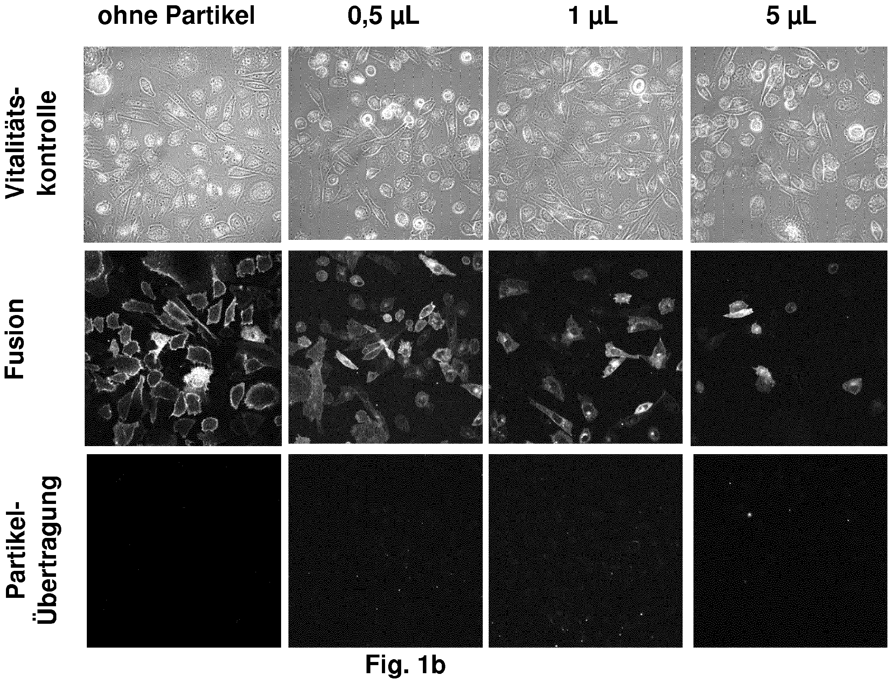

Fig. 1b illustriert die stark reduzierte Fusogenität klassischer Fusionssysteme bei ansteigender Konzentration geladener Partikel (positiv geladene Nanokugeln). Zur Überprüfung der Übertragungseffizienz geladener Partikel durch klassische, fusogene Liposome der Zusammensetzung: positiv geladenes Lipid (DOTAP), fusogenes Molekül (DiR) und neutrales Lipid (DOPE) im Gewichtsverhältnis 1:0,1:1, wurden 10 µl einer 3 mM Lösung verwendet und mit steigenden Konzentrationen an positiv geladenen Nanokugeln inkubiert. Die Inkubation erfolgte in 20 mM HEPES pH 7,4 für 10 min bei RT. Sofern nicht anders angegeben, wurden die Grundzusammensetzung der fusogenen Liposome (DOTAP / DiR / DOPE) auch für die nachfolgenden Ausführungsbeispiele und Abbildungen beibehalten.

Anschließend wurden die fusogenen Lipid-Kugel-Liposomen im Ultraschallbad bei 36 kHz und 200 W für 20 min bei RT behandelt, um fusogene Liposome mit einer mittleren Größe von etwa 340 nm zu erhalten. 10 µl der entstehenden fusogenen Lipid-Kugel-Liposome wurden mit PBS im Verhältnis 1:50 verdünnt und nochmals für 20 min unter gleichen Bedingungen wie zuvor im Ultraschallbad inkubiert, um eine möglichst hohe Anzahl fusogener Liposome zu erhalten. Die verdünnten Liposomen wurden anstelle des Zellkulturmediums zu adhärenten CHO (Chinese hamster ovary) Zellen gegeben, die am Tag zuvor in einer Dichte von 15.000 Zellen pro cm² ausgesät wurden, und für 20 min bei 37°C inkubiert.

Fusion wurde durch Austausch der Fusionslösung gegen Zellkulturmedium gestoppt. Direkt nach Beendigung der Fusionsreaktion wurde mittels Fluoreszenzmikroskopie die Fusionseffizienz (Mitte) als auch die funktionelle Übertragung an fluoreszenten Kugeln (unten) überprüft. Zu beachten ist die starke Reduktion der Fusogenität der Liposome mit steigender Konzentration an geladenen Partikeln. Gleichzeitig findet nahezu keine Übertragung geladener Partikel statt.

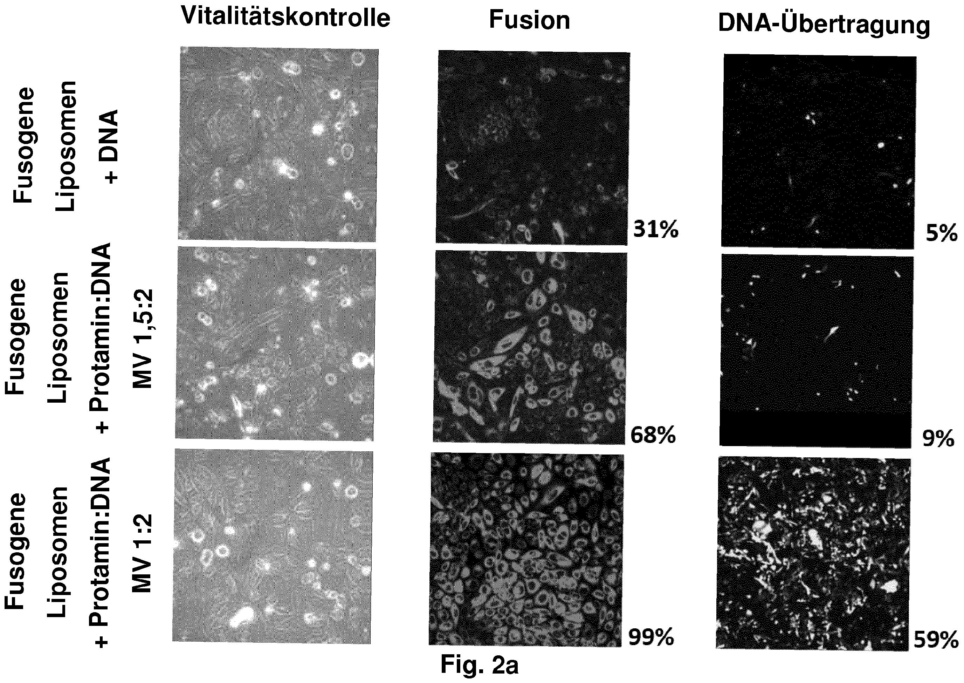

Fig. 2a illustriert den Einfluss der Neutralisierung geladener Moleküle (DNA) mittels Peptid auf die Fusogenität fusogener Liposomen sowie die Übertragungseffizienz. Zur Herstellung des Komplexes aus DNA und Protamin als Komplexbildner (Komplex A) wurden pro Ansatz 2 µg cDNA entweder ohne ("Fusogene Liposomen + DNA", oben) oder mit definierten Konzentrationen an polykationischer Substanz (Protamin) als neutralisierendem Agens im Gewichtsverhältnis (Protamin:DNA) 1,5:2 (Mitte) und 1:2 (unten) in die Fusionsreaktion eingesetzt. Als Lösungspuffer wurden 5 µl Tris-Puffer (Tris(hydroxymethyl)-aminomethan) verwendet (10 mM Tris, pH 7,5). Die Inkubation in Lösungspuffer erfolgte während einer 20-minütigen Inkubation bei RT. Während dieser Zeit wurden 10 µl einer 3 mM fusogenen Lipidmischung im Ultraschallbad bei 45 kHz und 70 W für 10 min bei RT suspendiert, um fusogene Liposome mit einer mittleren Größe von etwa 340 nm zu erhalten.

Die Lipidzusammensetzung der fusogenen Liposome bestand aus positiv geladenem Lipid, fusogenem Molekül und neutralem Lipid im Gewichtsverhältnis 1:0,1:1 in 20 mM HEPES, pH 7,4. Nach Beendigung der Inkubationszeiten wurde den fusogenen Liposomen 1 µl Tritonpuffer (10 µM Triton-X100, 10 µM NaCl, 2 µM TrisHCl pH 7,6) zugegeben und anschließend mit fertigem Komplex A zusammengegeben und für 20 min bei 45 kHz und RT im Ultraschallbad behandelt. 10 µl der entstehenden fusogenen Komplex-A-Liposomen wurden mit PBS im Verhältnis 1:50 verdünnt und nochmals für 20 min unter gleichen Bedingungen wie zuvor im Ultraschallbad inkubiert, um eine möglichst hohe Anzahl fusogener Liposome zu erhalten.

Die verdünnten Komplex-A-Liposomen wurden anstelle des Zellkulturmediums zu adhärenten CHO (Chinese hamster ovary) Zellen gegeben, die am Tag zuvor in einer Dichte von 15.000 Zellen pro cm² ausgesät wurden, und für 20 min bei 37°C inkubiert. Fusion wurde durch Austausch der Fusionslösung gegen Zellkulturmedium gestoppt. 24 Stunden nach Beendigung der Fusionsreaktion wurde mittels Fluoreszenzmikroskopie und Durchflusszytometrie die Fusionseffizienz als auch die funktionelle Übertragung des Plasmids mittels Proteinexpressionsanalyse überprüft. Die in der Durchflusszytometrie als positiv identifizierten Zellen (rot leuchtend durch das in der Fusionsmischung enthaltene rot leuchtende DiR sowie zusätzlich grün leuchtend nach Translation der eingebrachten DNA in grün fluoreszierendes Protein (GFP)) sind neben den Bildern in Prozent angeben.

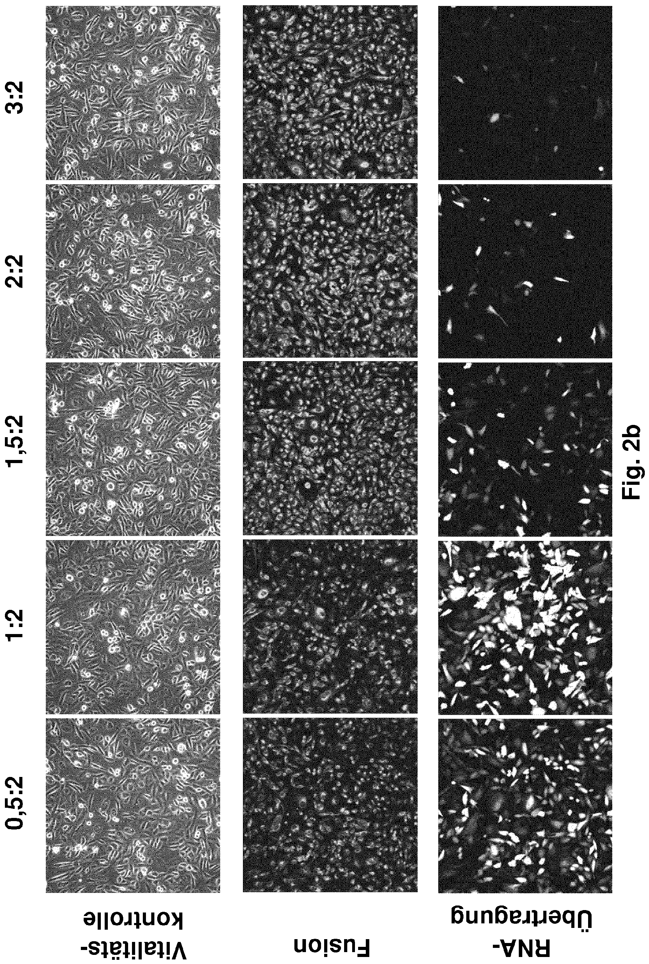

Fig. 2b illustriert den Einfluss der Neutralisierung geladener Moleküle (RNA) mittels Peptid auf die Fusogenität fusogener Liposomen sowie die Übertragungseffizienz. Zur Herstellung des Komplexes aus RNA und Komplexbildner (Komplex A) wurden pro Ansatz 2 µg RNA zur Expression von GFP (grün fluoreszierendes Protein) mit definierten Konzentrationen an neutralisierender polykationischer Substanz (Protamin) in den angegebenen Gewichtsverhältnissen (Protamin:RNA) in die Fusionsreaktion eingesetzt. Als Lösungspuffer wurden 5 µl Puffer verwendet (10 mM Tris, pH 7,5). Die Inkubation in Lösungspuffer erfolgte während einer 20-minütigen Inkubation bei RT. Während dieser Zeit wurden 10 µl einer 3 mM fusogenen Lipidmischung im Ultraschallbad bei 36 kHz und 70 W für 10 min bei RT suspendiert, um fusogene Liposome mit einer mittleren Größe von etwa 340 nm zu erhalten.

Die Lipidzusammensetzung der fusogenen Liposome bestand aus positiv geladenem Lipid, fusogenem Molekül und neutralem Lipid im Gewichtsverhältnis 1:0,1:1 in 20 mM HEPES, pH 7,4. Nach Beendigung der Inkubationszeiten wurden fusogene Liposome mit 100 µM NaCl versetzt und mit fertigem Komplex A für 20 min bei 36 kHz und RT im Ultraschallbad behandelt. 10 µl der entstehenden fusogenen Komplex-A-Liposomen wurden mit PBS im Verhältnis 1:50 verdünnt und nochmals für 20 min unter gleichen Bedingungen wie zuvor im Ultraschallbad inkubiert, um eine möglichst hohe Anzahl fusogener Liposome zu erhalten.

Die verdünnten Komplex-A-Liposomen wurden anstelle des Zellkulturmediums zu adhärenten CHO (Chinese hamster ovary) Zellen gegeben, die am Tag zuvor in einer Dichte von 15.000 Zellen pro cm² ausgesät wurden und für 20 min bei 37°C inkubiert. Fusion wurde durch Austausch der Fusionslösung gegen Zellkulturmedium gestoppt. 3 Stunden nach Beendigung der Fusionsreaktion wurde mittels Fluoreszenzmikroskopie die Fusionseffizienz als auch die Expression der mRNA mittels Proteinexpressionsanalyse überprüft. Deutlich sichtbar ist die besonders vorteilhafte RNA-Übertragung bei 0,5:2 sowie 1:2 Verhältnissen von RNA : Protamin, während die Fusionseffizienz kaum durch unterschiedliche Mischungsverhältnisse beeinflusst wird.

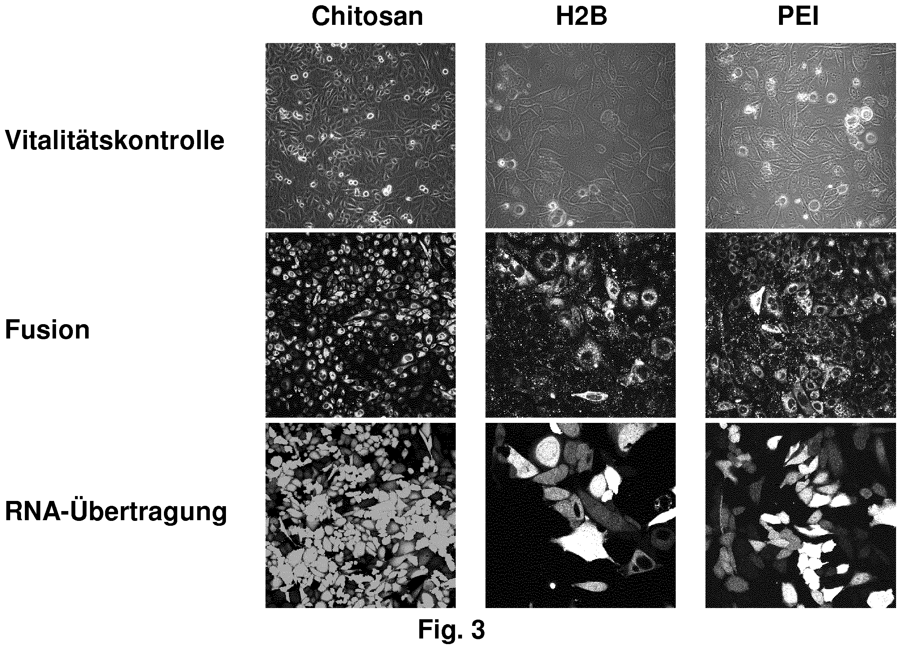

Fig. 3 illustriert den Einfluss der Neutralisierung geladener Moleküle (RNA) mittels polyprotonierter Polymere auf Fusogenität und Übertragungseffizienz. Zur Herstellung des Komplexes aus DNA und Komplexbildner (Komplex A) wurden pro Ansatz 2 µg DNA zur Expression von GFP (grün fluoreszierendes Protein) mit 1 µg Poly-Ethylenimin (PEI), 1 µg H2B Histonprotein bzw. 0,5 µg Chitosan als polykationische Substanz als Alternative zu Protamin inkubiert. Als Lösungspuffer wurden 5 µl Puffer (10 mM Tris, pH 7,5) verwendet. Die Inkubation in Lösungspuffer erfolgte während einer 20-minütigen Inkubation bei RT. Während dieser Zeit wurden 10 µl einer 3 mM fusogenen Lipidmischung im Ultraschallbad bei 45 kHz und 70 W für 10 min bei RT suspendiert, um fusogene Liposome mit einer mittleren Größe von etwa 340 nm zu erhalten.

Die Lipidzusammensetzung der fusogenen Liposome bestand aus positiv geladenem Lipid, fusogenem Molekül und neutralem Lipid im Gewichtsverhältnis 2:0,2:1 in 20 mM HEPES, pH 7,4. Nach Beendigung der Inkubationszeiten wurden fusogene Liposome mit 50 µM NaCl versetzt und mit fertigem Komplex A für 20 min bei 45 kHz und RT im Ultraschallbad behandelt. 10 µl der entstehenden fusogenen Komplex-A-Liposomen wurden mit PBS im Verhältnis 1:50 verdünnt und nochmals für 20 min unter gleichen Bedingungen wie zuvor im Ultraschallbad inkubiert, um eine möglichst hohe Anzahl fusogener Liposome zu erhalten.

Die verdünnten Komplex-A-Liposomen wurden anstelle des Zellkulturmediums zu adhärenten CHO (Chinese hamster ovary) Zellen gegeben, die am Tag zuvor in einer Dichte von 15.000 Zellen pro cm² ausgesät wurden, und für 20 min bei 37°C inkubiert. Fusion wurde durch Austausch der Fusionslösung gegen Zellkulturmedium gestoppt. 3 Stunden nach Beendigung der Fusionsreaktion wurde mittels Fluoreszenzmikroskopie die Fusionseffizienz als auch die funktionelle Expression der mRNA mittels Proteinexpressionsanalyse überprüft. Zu beachten ist die erfolgende Fusion sowie funktionelle Übertragung der geladenen Moleküle auch bei PEI bzw. H2B Komplexierung.

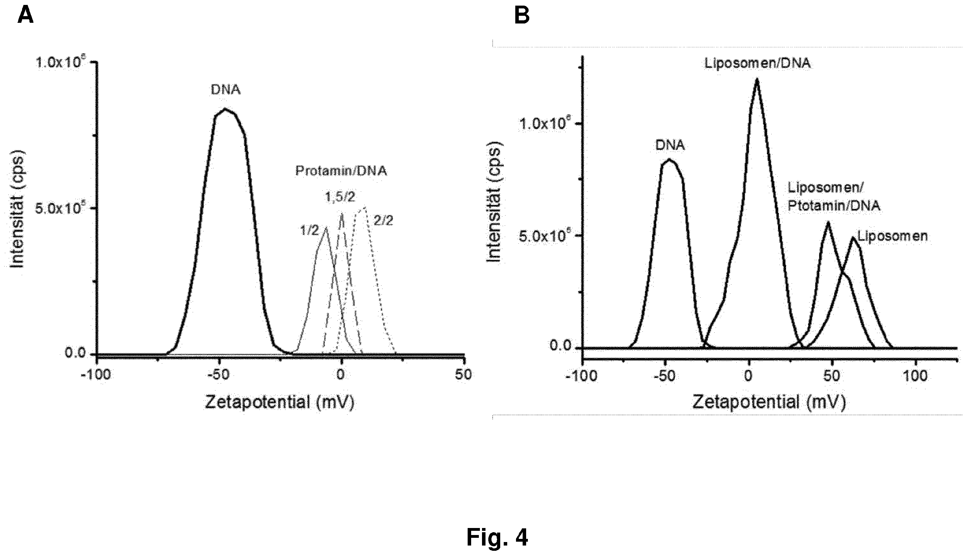

Fig. 4 illustriert die Zetapotentialverschiebung in Abhängigkeit der Komplexzusammensetzung. Das Zetapotential von DNA wurde für die linke Grafik A dadurch bestimmt, dass 2 µg DNA in einer Wasser/PBS-Mischung (950 µl destilliertes Wasser und 50 µl PBS) aufgenommen wurde. Es zeigt sich ein Zetapotential um die -50 mV.

Wenn die identische Menge von DNA in unterschiedlichen Verhältnissen mit Protamin für 0,5 h bei RT komplexiert wurde (1; 1,5 und 2 µg Protamin entspricht Protamin/DNA-Verhältnissen von 1/2; 1,5/2 und 2/2) wird das Zetapotential der Komplexe deutlich beeinflusst. Mit steigender Protaminkonzentration wird das Zetapotential der Komplexe in positive Bereiche verschoben. Das Protamin/DNA―Verhältnis wird vorzugsweise so eingestellt, dass die Zetapotentiale der Komplexe im Vergleich zu DNA deutlich reduziert werden und trotzdem die Komplexe in idealer Weise noch mit den positiv geladenen Liposomen elektrostatisch wechselwirken (deren Zetapotential verbleibt damit im negativen Bereich). Diesen Kriterien entspricht in diesem Beispiel der Komplex mit dem 1/2 Protamin/DNA-Verhältnis.

Um die Abhängigkeit von Transfektionseffizienz und Zetapotential zu demonstrieren, wurden für die linke Grafik B Liposomen wie vorher beschrieben mit einer Konzentration von 3 mM in 20 mM HEPES-Puffer hergestellt und in einer Wasser/PBS-Mischung (940 µl destilliertes Wasser und 50 µl PBS) 1/100 verdünnt. 10 µl dieser Liposomen wurden vor der Verdünnung entweder nur mit 2 µg DNA oder mit 2 µg DNA neutralisiert mit 1 µg Protamin (1/2) inkubiert und, wie 2 µg DNA, 1/100 verdünnt. Die Liposomen zeigen ein positives Zetapotential (ca. 60 mV), während sich die DNA im negativen Bereich bewegte (-50 mV).

Bei Inkubation der Liposomen mit Protamin-komplexierter DNA wurde deren positives Zetapotential deutlich weniger reduziert (50 mV) als im Fall ohne Protamin (10 mV). Ein Zetapotential im Bereich von 50 mV ist damit noch ausreichend für eine erfolgreiche Fusion und DNA-Transfer, während ein niedrigeres Zetapotenzial der Komplexe nicht zur Fusion führt.

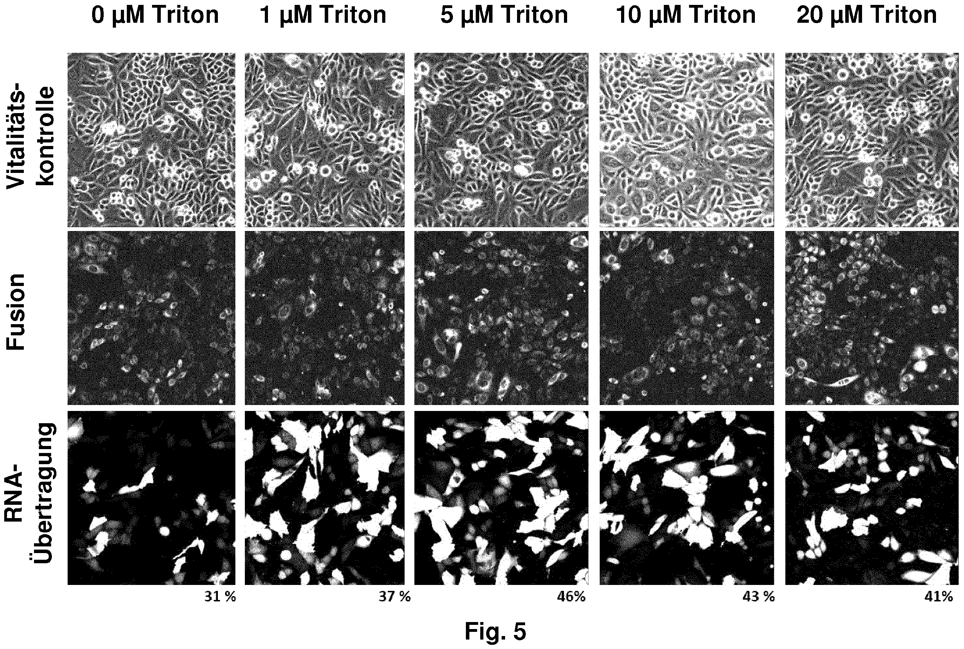

Fig. 5 illustriert den Einfluss der Membran-Destabilisierung auf Fusogenität und Übertragung geladener Moleküle. Zur Herstellung des Komplexes aus DNA und Komplexbildner (Komplex A) wurden pro Ansatz 2 µg cDNA zur Expression von GFP (grün fluoreszierendes Protein) mit polykationischer Substanz (Protamin) im Gewichtsverhältnis 2:1 inkubiert. Als Lösungspuffer wurden 5 µl Puffer verwendet (10 mM Tris, pH 7,5). Vor Inkubation für 20 min bei RT wurde den Ansätzen Triton X-100 in den Konzentrationen 0, 1, 5, 10 und 20 µM zugesetzt. Während dieser Zeit wurden 10 µl einer 3 mM fusogenen Lipidmischung im Ultraschallbad bei 45 kHz und 70 W für 10 min bei RT suspendiert, um fusogene Liposome mit einer mittleren Größe von etwa 340 nm zu erhalten.

Die Lipidzusammensetzung der fusogenen Liposome bestand aus positiv geladenem Lipid, fusogenem Molekül und neutralem Lipid im Gewichtsverhältnis 1:0,1:1 in 20 mM HEPES, pH 7,4. Nach Beendigung der Inkubationszeiten wurden fusogene Liposome mit fertigem Komplex A inkubiert und für 20 min bei 45 kHz und RT im Ultraschallbad behandelt. 10 µl der entstehenden fusogenen Komplex-A-Liposomen wurden mit PBS im Verhältnis 1:50 verdünnt und nochmals für 20 min unter gleichen Bedingungen wie zuvor im Ultraschallbad inkubiert, um eine möglichst hohe Anzahl fusogener Liposome zu erhalten.

Die verdünnten Komplex-A-Liposomen wurden anstelle des Zellkulturmediums zu adhärenten HeLa Zellen gegeben, die am Tag zuvor in einer Dichte von 15.000 Zellen pro cm² ausgesät wurden, und für 20 min bei 37°C inkubiert. HeLa Zellen wurden verwendet, da diese Zellen nur eine erniedrigte Fusionseffizienz und DNA-Übertragungsrate aufweisen. Fusion wurde durch Austausch der Fusionslösung gegen Zellkulturmedium gestoppt. 24 Stunden nach Beendigung der Fusionsreaktion wurde mittels Fluoreszenzmikroskopie und Durchflusszytometrie die Fusionseffizienz als auch die funktionelle Übertragung des Plasmids mittels Proteinexpressionsanalyse überprüft. Die in der Durchflusszytometrie als positiv identifizierten Zellen sind in Prozent angeben. Zu beachten ist, dass Zellen mit natürlicherweise erniedrigter Fusionseffizienz in Anwesenheit geringer Konzentrationen an Membran-destabilisierenden Molekülen sowohl fusioniert als auch mit geladenen Molekülen beladen werden können.

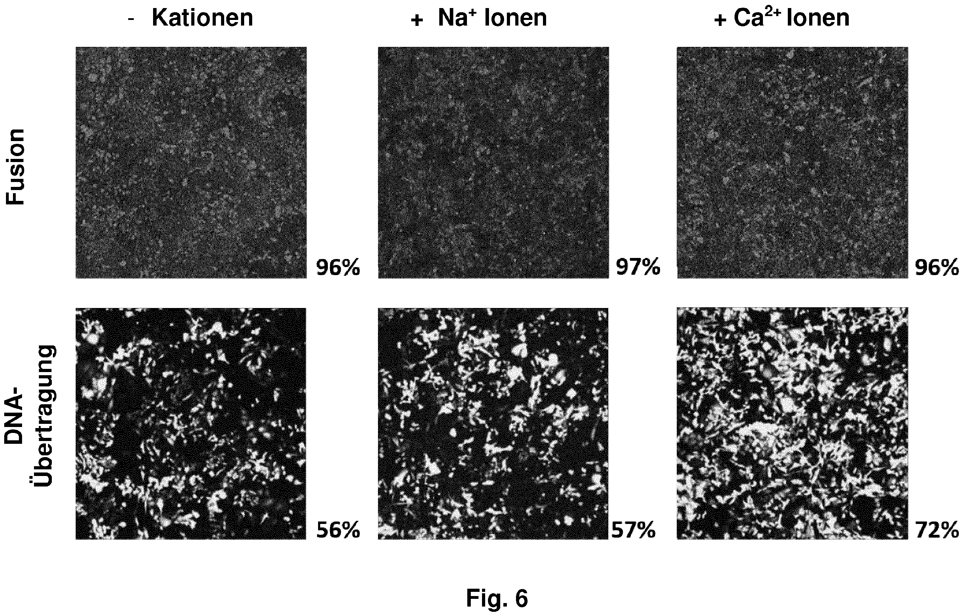

Fig. 6 illustriert den Einfluss zusätzlicher Kationen auf Fusogenität und Übertragung geladener Moleküle. Zur Herstellung des Komplexes aus DNA und Komplexbildner (Komplex A) wurden pro Ansatz 2 µg cDNA zur Expression von GFP (grün fluoreszierendes Protein) mit polykationischer Substanz (Protamin) im Gewichtsverhältnis 2:1 inkubiert. Als Lösungspuffer wurden 5 µl Puffer verwendet (10 mM Tris, pH 7,5). Zusätzlich wurden in einen Ansatz Na⁺-Ionen zugegeben (100 mM NaCl). Die Inkubation in Lösungspuffer erfolgte während einer 20-minütigen Inkubation bei RT. Während dieser Zeit wurden 10 µl einer 3 mM fusogenen Lipidmischung im Ultraschallbad bei 36 kHz und 70 W für 10 min bei RT suspendiert, um fusogene Liposome mit einer mittleren Größe von etwa 340 nm zu erhalten.

Die Lipidzusammensetzung der fusogenen Liposome bestand aus positiv geladenem Lipid, fusogenem Molekül und neutralem Lipid im Gewichtsverhältnis 1:0,1:1 in 20 mM HEPES, pH 7,4. Nach Beendigung der Inkubationszeiten wurden fusogene Liposome mit fertigem Komplex A inkubiert und für 20 min bei 45 kHz und RT im Ultraschallbad behandelt. 10 µl der entstehenden fusogenen Komplex-A-Liposomen wurden mit PBS im Verhältnis 1:50 verdünnt. In einen Ansatz, der zuvor nur in 20 mM HEPES als Lösungspuffer inkubiert worden war, wurden zu diesem Zeitpunkt noch Ca²⁺ Ionen (20 µM CaCl₂) zugesetzt. Anschließend wurden alle Ansätze nochmals für 20 min unter gleichen Bedingungen wie zuvor im Ultraschallbad inkubiert, um eine möglichst hohe Anzahl fusogener Liposome zu erhalten.

Die verdünnten Komplex-A-Liposomen wurden anstelle des Zellkulturmediums zu adhärenten CHO (Chinese hamster ovary) Zellen gegeben, die am Tag zuvor in einer Dichte von 15.000 Zellen pro cm² ausgesät wurden, und für 20 min bei 37°C inkubiert. Fusion wurde durch Austausch der Fusionslösung gegen Zellkulturmedium gestoppt. 24 Stunden nach Beendigung der Fusionsreaktion wurde mittels Fluoreszenzmikroskopie und Durchflusszytometrie die Fusionseffizienz als auch die funktionelle Übertragung des Plasmids mittels Proteinexpressionsanalyse überprüft. Die in der Durchflusszytometrie als positiv identifizierten Zellen sind in Prozent angeben. Während die Fusion selbst nicht durch zusätzliche Kationen verändert ist, zeigen diese eine positive Wirkung auf die funktionelle Übertragung geladener Moleküle.

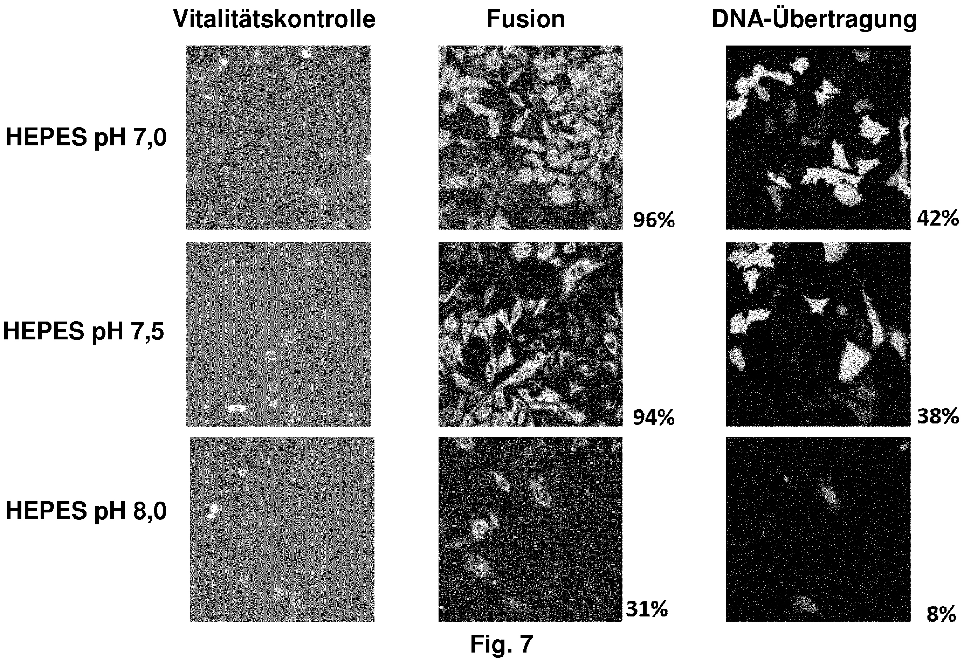

Fig. 7 illustriert den Einfluss des pH-Wertes des Liposomenpuffers auf Fusogenität und Übertragung geladener Moleküle. Zur Herstellung des Komplexes aus DNA und Komplexbildner (Komplex A) wurden pro Ansatz 2 µg cDNA zur Expression von GFP (grün fluoreszierendes Protein) mit polykationischer Substanz (Protamin) im Gewichtsverhältnis 2:1 inkubiert. Als Lösungspuffer wurden 5 µl Puffer verwendet (20 mM HEPES, 100 mM NaCl). Pro Ansatz wurde der pH-Wert des Puffers im Bereich von 7,0 über 7,4 bis 8,0 variiert. Die Inkubation in Lösungspuffer erfolgte während einer 20-minütigen Inkubation bei RT. Während dieser Zeit wurden 10 µl einer 3 mM fusogenen Lipidmischung im Ultraschallbad bei 36 kHz und 70 W für 10 min bei RT suspendiert, um fusogene Liposome mit einer mittleren Größe von etwa 340 nm zu erhalten.

Die Lipidzusammensetzung der fusogenen Liposome bestand aus positiv geladenem Lipid, fusogenem Molekül und neutralem Lipid im Gewichtsverhältnis 1:0,1:1 in 20 mM HEPES, pH 7,4. Nach Beendigung der Inkubationszeiten wurden fusogene Liposome mit 1 µl einer 100 mM Lösung NaCl versetzt und mit fertigem Komplex A für 20 min bei 45 kHz und RT im Ultraschallbad behandelt. 10 µl der entstehenden fusogenen Komplex-A-Liposomen wurden mit PBS im Verhältnis 1:50 verdünnt und nochmals für 20 min unter gleichen Bedingungen wie zuvor im Ultraschallbad inkubiert, um eine möglichst hohe Anzahl fusogener Liposome zu erhalten.

Die verdünnten Komplex-A-Liposomen wurden anstelle des Zellkulturmediums zu adhärenten CHO (Chinese hamster ovary) Zellen gegeben, die am Tag zuvor in einer Dichte von 15.000 Zellen pro cm² ausgesät wurden, und für 20 min bei 37°C inkubiert. Fusion wurde durch Austausch der Fusionslösung gegen Zellkulturmedium gestoppt. 24 Stunden nach Beendigung der Fusionsreaktion wurde mittels Fluoreszenzmikroskopie und Durchflusszytometrie die Fusionseffizienz als auch die funktionelle Übertragung des Plasmids mittels Proteinexpressionsanalyse überprüft. Die in der Durchflusszytometrie als positiv identifizierten Zellen sind in Prozent angeben. Deutlich zu erkennen ist ein pH-Optimum im Bereich um 7 bis 7,4. Höhere pH-Werte können sowohl die Fusion als auch die Übertragung geladener Moleküle behindern.

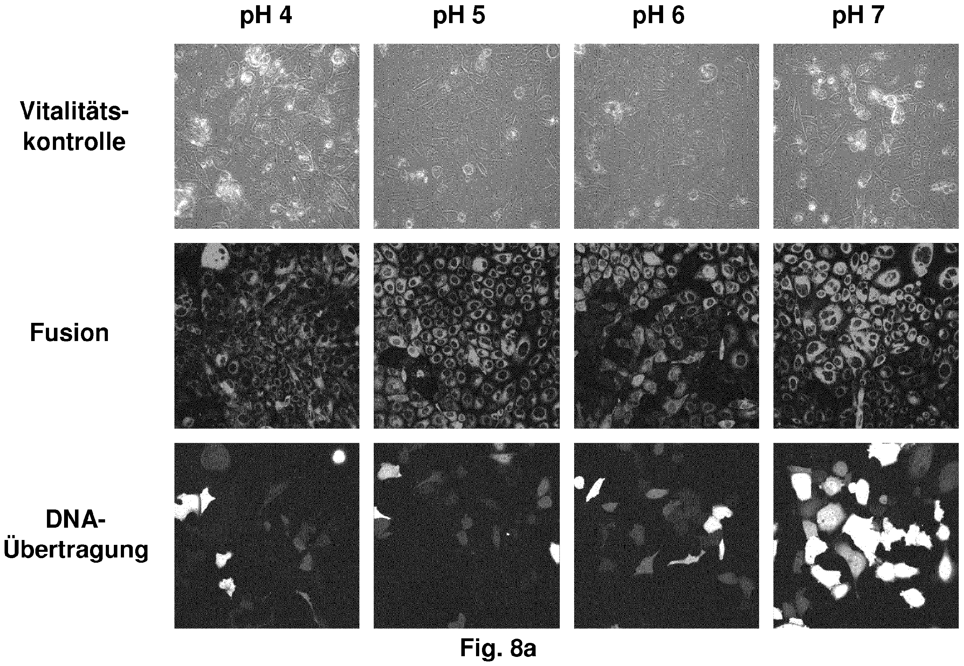

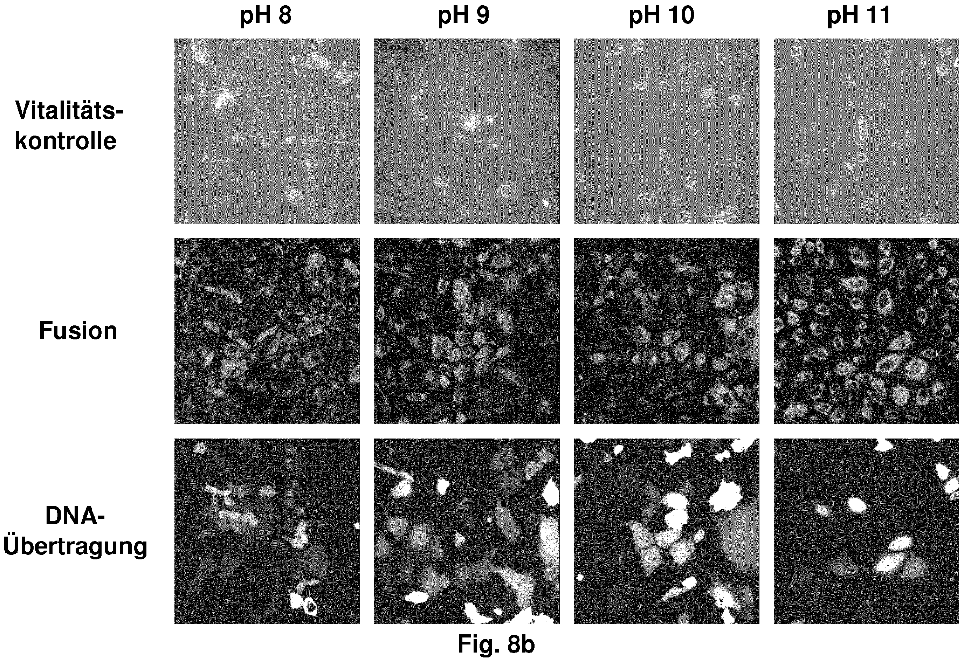

Fig. 8a und 8b illustrieren den Einfluss des pH-Wertes des Verdünnungspuffers (PBS) nach Erzeugung des fusogenen Komplex-A-Liposoms auf Fusogenität und Übertragung geladener Moleküle Zur Herstellung des Komplexes aus DNA und Komplexbildner (Komplex A) wurden pro Ansatz 2 µg cDNA zur Expression von GFP (grün fluoreszierendes Protein) mit polykationischer Substanz (Protamin) im Gewichtsverhältnis 2:1 inkubiert. Als Lösungspuffer wurden 5 µl Puffer verwendet (10 mM Tris, pH 7,5). Die Inkubation in Lösungspuffer erfolgte für 20 min bei RT. Während dieser Zeit wurden 10 µl einer 3 mM fusogenen Lipidmischung im Ultraschallbad bei 36 kHz und 200 W für 10 min bei RT suspendiert, um fusogene Liposome mit einer mittleren Größe von etwa 340 nm zu erhalten.

Die Lipidzusammensetzung der fusogenen Liposome bestand aus positiv geladenem Lipid, fusogenem Molekül und neutralem Lipid im Gewichtsverhältnis 1:0,1:1 in 20 mM HEPES, pH 7,4. Nach Beendigung der Inkubationszeiten wurde den fusogenen Liposomen 1 µl einer 100 mM Lösung NaCl zugegeben mit fertigem Komplex A und für 20 min bei 36 kHz und RT im Ultraschallbad inkubiert. 10 µl der entstehenden fusogenen Komplex-A-Liposomen wurden mit PBS im Verhältnis 1:50 verdünnt. Der pH-Wert des PBS-Puffers wurde hierbei im Bereich von pH 4 bis pH 11 variiert. Fig. 8a gibt den pH-Bereich von 4 bis 7 wieder und Fig. 8b stellt den pH-Bereich von pH 8 bis 11 dar. Nach Verdünnung wurden alle Ansätze nochmals für 20 min unter gleichen Bedingungen wie zuvor im Ultraschallbad inkubiert, um eine möglichst hohe Anzahl fusogener Liposome zu erhalten.

Die verdünnten Komplex-A-Liposomen wurden anstelle des Zellkulturmediums zu adhärenten CHO (Chinese hamster ovary) Zellen gegeben, die am Tag zuvor in einer Dichte von 15.000 Zellen pro cm² ausgesät wurden, und für 20 min bei 37°C inkubiert. Fusion wurde durch Austausch der Fusionslösung gegen Zellkulturmedium gestoppt. 24 Stunden nach Beendigung der Fusionsreaktion wurde mittels Fluoreszenzmikroskopie sowohl die Fusionseffizienz als auch die funktionelle Übertragung des Plasmids mittels Proteinexpressionsanalyse überprüft. Besonders vorteilhaft ist der Bereich um pH 7 bis 9 für die funktionelle Übertragung geladener Moleküle. Hierbei ist die Übertragung auch noch bei höheren pH-Werten möglich, doch geht diese mit dann steigendem osmotischem Stress für die Zellen einher. Die Fusion selbst ist im getesteten pH-Bereich nahezu unverändert.

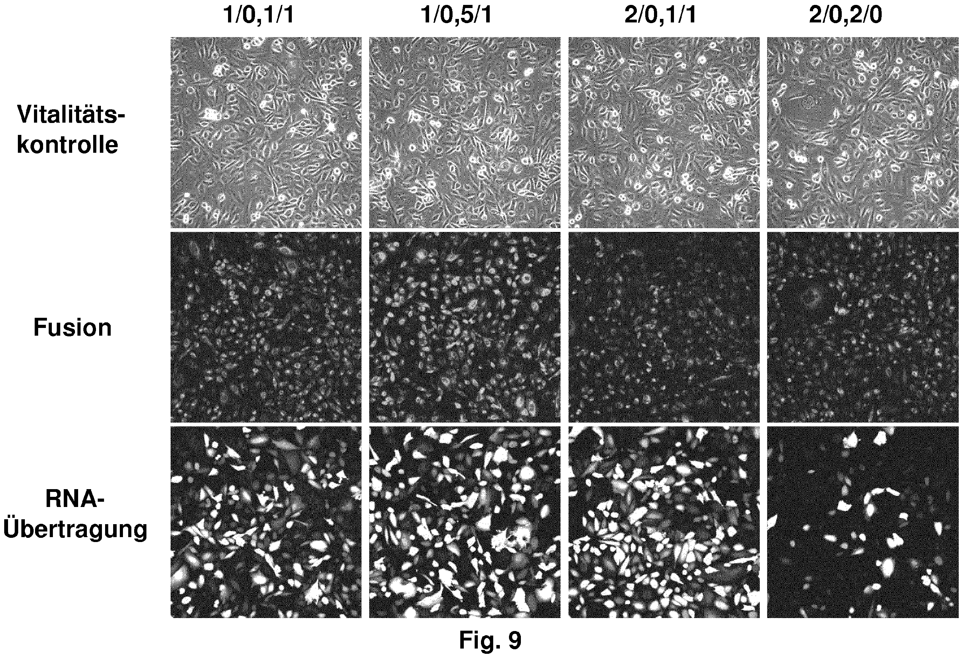

Fig. 9 illustriert die Abhängigkeit der Fusogenität und Übertragbarkeit geladener Moleküle von der Zusammensetzung der fusogenen Liposomen. Zur Herstellung des Komplexes aus mRNA und Komplexbildner (Komplex A) wurden pro Ansatz 2 µg mRNA zur Expression von GFP (grün fluoreszierendes Protein) mit polykationischer Substanz (Protamin) im Gewichtsverhältnis 2:1 inkubiert. Als Lösungspuffer wurden 5 µl Puffer verwendet (10 mM Tris, pH 7,5). Die Inkubation in Lösungspuffer erfolgte während einer 20-minütigen Inkubation bei RT. Während dieser Zeit wurden 10 µl einer 3 mM fusogenen Lipidmischung im Ultraschallbad bei 36 kHz und 200 W für 10 min bei RT suspendiert, um fusogene Liposome mit einer mittleren Größe von etwa 340 nm zu erhalten.

Die Lipidzusammensetzung der fusogenen Liposome wurde pro Ansatz derart variiert, dass für die Bestandteile positiv geladenes Lipid : fusogenes Molekül: neutrales Lipid die in der Abbildung angegebenen Gewichtsverhältnisse 1:0,1:1, 1:1:0,5, 2:1:0,1 und 2:0,2:0 in 20 mM HEPES, pH 7,4 verwendet wurden. Nach Beendigung der Inkubationszeiten wurden die entstandenen fusogenen Liposome mit 1 µl einer 100 mM NaCl Lösung versetzt und jeweils mit fertigem Komplex A für 20 min bei 36 kHz und RT im Ultraschallbad inkubiert. 10 µl der entstandenen fusogenen Komplex-A-Liposomen wurden mit PBS im Verhältnis 1:50 verdünnt. Nach Verdünnung wurden alle Ansätze nochmals für 20 min unter gleichen Bedingungen wie zuvor im Ultraschallbad inkubiert, um eine möglichst hohe Anzahl fusogener Liposome zu erhalten.

Die verdünnten Komplex-A-Liposomen wurden anstelle des Zellkulturmediums zu adhärenten CHO (Chinese hamster ovary) Zellen gegeben, die am Tag zuvor in einer Dichte von 15.000 Zellen pro cm² ausgesät wurden, und für 20 min bei 37°C inkubiert. Fusion wurde durch Austausch der Fusionslösung gegen Zellkulturmedium gestoppt. 3 Stunden nach Beendigung der Fusionsreaktion wurde mittels Fluoreszenzmikroskopie sowohl die Fusionseffizienz als auch die funktionelle Expression der mRNA mittels Proteinexpressionsanalyse überprüft. Deutlich zu erkennen ist, dass die in erfindungsgemäße Vorgehensweise für alle getesteten Lipidverhältnisse sowohl Fusion als auch eine Übertragung geladener Moleküle ermöglicht.

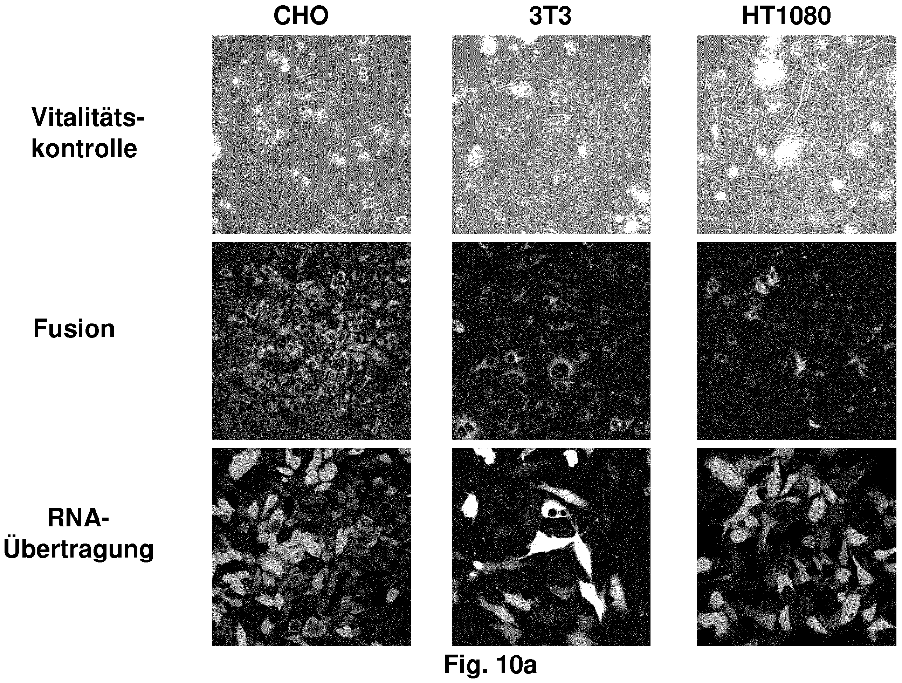

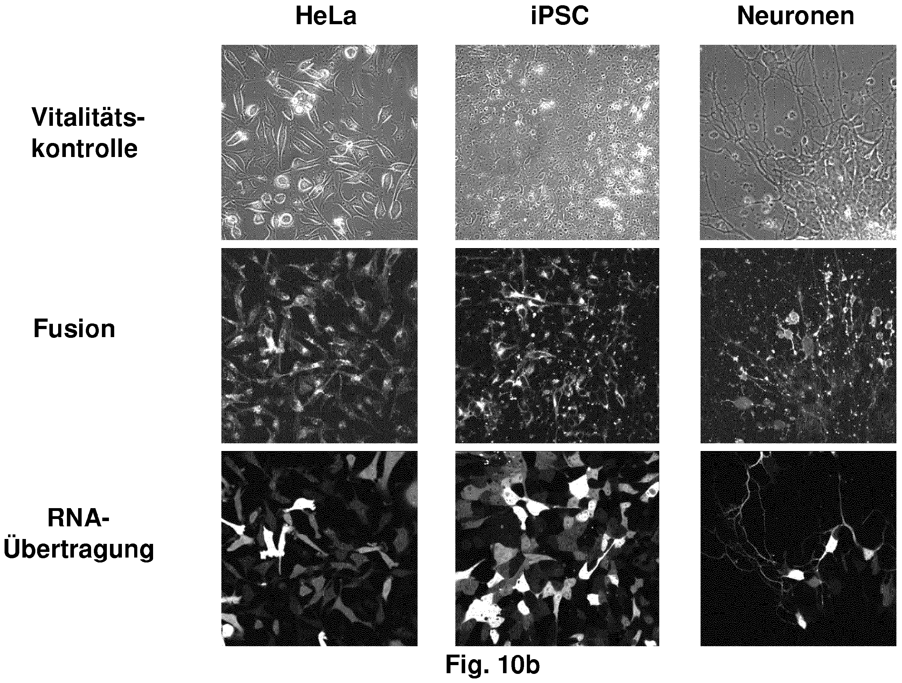

Fig. 10a und 10b illustrieren die universelle Übertragbarkeit geladener Moleküle in unterschiedliche Zelllinien und Primärzellen mittels erfindungsgemäß erzeugter Fusionsmischung. Zur Herstellung des Komplexes aus mRNA und Komplexbildner (Komplex A) wurden pro Ansatz 2 µg mRNA zur Expression von GFP (grün fluoreszierendes Protein) mit polykationischer Substanz (Protamin) im Gewichtsverhältnis 2:1 inkubiert. Als Lösungspuffer wurden 5 µl Puffer verwendet 10 mM Tris, pH 7,5). Die Inkubation in Lösungspuffer erfolgte während einer 20-minütigen Inkubation bei RT. Während dieser Zeit wurden 10 µl einer 3 mM fusogenen Lipidmischung im Ultraschallbad bei 36 kHz und 200 W für 10 min bei RT suspendiert, um fusogene Liposome mit einer mittleren Größe von etwa 340 nm zu erhalten.

Die Lipidzusammensetzung der fusogenen Liposome bestand aus positiv geladenem Lipid, fusogenem Molekül und neutralem Lipid im Gewichtsverhältnis 1:0,1:1 in 20 mM HEPES, pH 7,4. Nach Beendigung der Inkubationszeiten wurden die entstandenen fusogenen Liposome mit 1 µl einer 100 mM NaCl Lösung versetzt und mit fertigem Komplex A für 20 min bei 36 kHz und RT im Ultraschallbad inkubiert. 10 µl der entstandenen fusogenen Komplex-A-Liposomen wurden mit PBS im Verhältnis 1:50 verdünnt. Nach Verdünnung wurden alle Ansätze nochmals für 20 min unter gleichen Bedingungen wie zuvor im Ultraschallbad inkubiert, um eine möglichst hohe Anzahl fusogener Liposome zu erhalten.

Die verdünnten Komplex-A-Liposomen wurden anstelle des Zellkulturmediums zu unterschiedlichen, in der Figur angegebenen Zelllinien und Primärzellen (CHO (Chinese hamster ovary), 3T3, HT1080, HeLa, iPSC, primäre cortikale Neuronen) gegeben, die am Tag zuvor in einer Dichte von 15.000 Zellen pro cm² ausgesät wurden. Abhängig vom Zelltyp und damit zelltyp-spezifischen Fusionseigenschaften wurden diese für 15 bis 30 min bei 37°C mit der Fusionslösung inkubiert (CHO = 15 min, Fig. 10a; 3T3 = 15 min, Fig. 10a; HT1080 = 30 min, Fig. 10a; HeLa = 30 min, Fig. 10b; iPSC = 25 min, Fig. 10b; Neuronen = 20 min, Fig. 10b). Fusion wurde durch Austausch der Fusionslösung gegen Zellkulturmedium gestoppt. 3 Stunden nach Beendigung der Fusionsreaktion wurde mittels Fluoreszenzmikroskopie sowohl die Fusionseffizienz als auch die funktionelle Expression der mRNA mittels Proteinexpressionsanalyse überprüft. Deutlich zu erkennen ist, dass in allen Fällen Fusion und DNA-Übertragung stattgefunden hat und damit das erfindungsgemäße Verfahren zur Herstellung einer Fusionsmischung universell für tierische Zelllinien und Primärzellen einsetzbar ist.

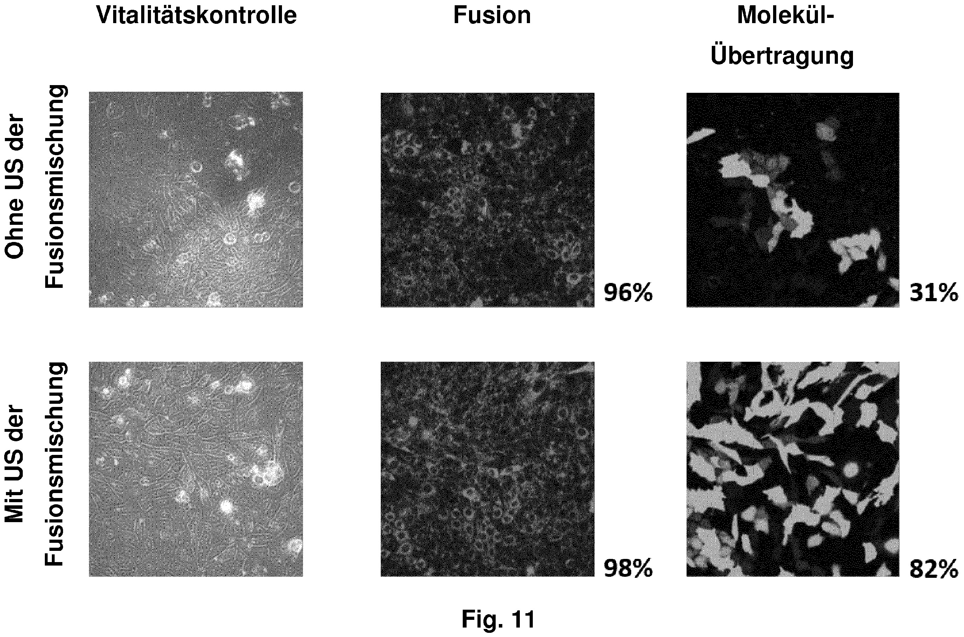

Fig. 11 illustriert den Einfluss zusätzlicher Ultraschallbehandlung auf Fusogenität und Übertragung geladener Moleküle. Zur Herstellung des Komplexes aus DNA und Komplexbildner (Komplex A) wurden pro Ansatz 2 µg cDNA zur Expression von GFP (grün fluoreszierendes Protein) mit polykationischer Substanz (Protamin) im Gewichtsverhältnis 2:1 inkubiert. Als Lösungspuffer wurden 5 µl Puffer verwendet (10 mM Tris, pH 7,5). Die Inkubation in Lösungspuffer erfolgte für 20 min bei RT. Während dieser Zeit wurden 10 µl einer 3 mM fusogenen Lipidmischung im Ultraschallbad bei 36 kHz und 200 W für 10 min bei RT suspendiert, um fusogene Liposome mit einer mittleren Größe von etwa 340 nm zu erhalten.

Die Lipidzusammensetzung der fusogenen Liposome bestand aus positiv geladenem Lipid, fusogenem Molekül und neutralem Lipid im Gewichtsverhältnis 1:0,1:1 in 20 mM HEPES, pH 7,4. Nach Beendigung der Inkubationszeiten wurden fusogene Liposome mit 1 µl einer 100 mM NaCl und 0,5 µM Triton X-100 Lösung versetzt und mit fertigem Komplex A für 20 min bei 36 kHz und RT im Ultraschallbad inkubiert. 10 µl der entstehenden fusogenen Komplex-A-Liposomen wurden mit PBS im Verhältnis 1:50 verdünnt. Während klassische Fusionsliposomen zur Übertragung ungeladener Moleküle die verdünnte Fusionslösung ohne weitere Ultraschallbehandlung direkt mit den zu fusionierenden Zellen inkubieren (Ohne US), wurde ein Ansatz nach Verdünnung nochmals für 20 min unter gleichen Bedingungen wie zuvor im Ultraschallbad (Mit US) inkubiert.

Die verdünnten Komplex-A-Liposomen wurden nachfolgend anstelle des Zellkulturmediums zu adhärenten CHO (Chinese hamster ovary) Zellen gegeben, die am Tag zuvor in einer Dichte von 15.000 Zellen pro cm² ausgesät wurden, und für 20 min bei 37°C inkubiert. Fusion wurde durch Austausch der Fusionslösung gegen Zellkulturmedium gestoppt. 24 Stunden nach Beendigung der Fusionsreaktion wurde mittels Fluoreszenzmikroskopie und Durchflusszytometrie die Fusionseffizienz als auch die funktionelle Übertragung des Plasmids mittels Proteinexpressionsanalyse überprüft. Die in der Durchflusszytometrie als positiv identifizierten Zellen sind in Prozent angeben. Zu erkennen ist eine weitere Verbesserung der funktionellen Übertragung geladener Moleküle bei Durchführung des zusätzlichen Ultraschallschrittes. Die Fusion selbst bleibt durch die Ultraschallbehandlung nahezu unverändert.

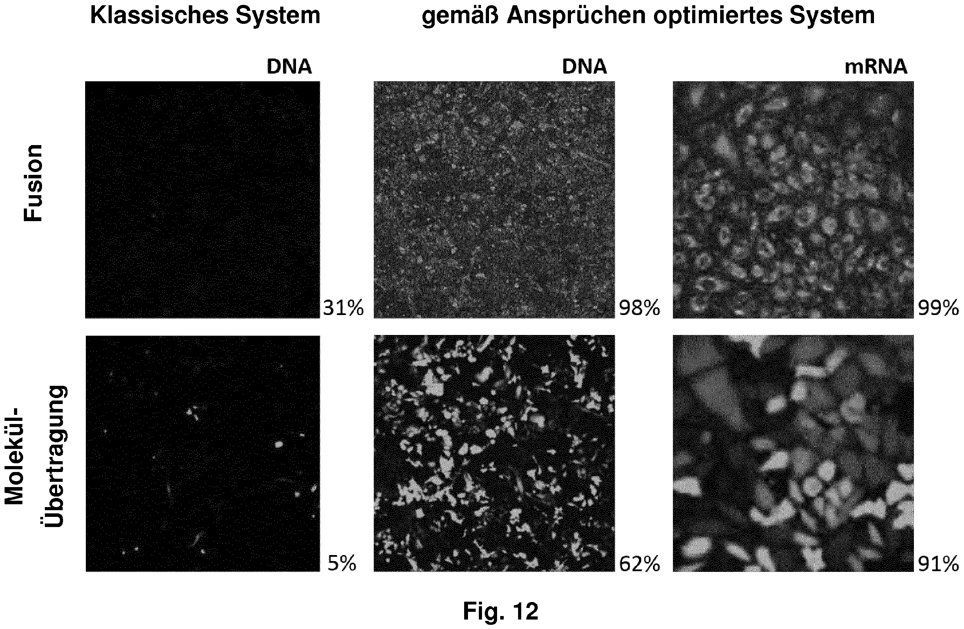

Fig. 12 illustriert herkömmliche fusogene Liposomen und ein erfindungsgemäßes Fusionssystem im direkten Vergleich. Zur Herstellung des Komplexes aus DNA und Komplexbildner bzw. RNA und Komplexbildner (Komplex A) wurden 2 µg cDNA bzw. 2 µg mRNA jeweils zur Expression von GFP (grün fluoreszierendes Protein) mit polykationischer Substanz (Protamin) im Gewichtsverhältnis 2:1 inkubiert. Als Lösungspuffer wurden 5 µl Puffer verwendet (10 mM Tris, pH 7,5). Die Inkubation in Lösungspuffer erfolgte für 20 min bei RT. Während dieser Zeit wurden 10 µl einer 3 mM fusogenen Lipidmischung im Ultraschallbad bei 36 kHz und 200 W für 10 min bei RT suspendiert, um fusogene Liposome mit einer mittleren Größe von etwa 340 nm zu erhalten.

Die Lipidzusammensetzung der fusogenen Liposome bestand aus positiv geladenem Lipid, fusogenem Molekül und neutralem Lipid im Gewichtsverhältnis 1:0,1:1 in 20 mM HEPES, pH 7,4. Nach Beendigung der Inkubationszeiten wurden fusogene Liposome mit 1 µl einer 100 mM NaCl und 0,5 µM Triton X-100 Lösung versetzt und mit fertigem Komplex A für 20 min bei 36 kHz und RT im Ultraschallbad inkubiert. 10 µl der entstehenden fusogenen Komplex-A-Liposomen wurden mit PBS im Verhältnis 1:50 verdünnt und nochmals für 5 min bei gleichen Parametern im Ultraschallbad behandelt..

Die verdünnten Komplex-A-Liposomen wurden nachfolgend anstelle des Zellkulturmediums zu adhärenten CHO (Chinese hamster ovary) Zellen gegeben, die zuvor in einer Dichte von 15.000 Zellen pro cm² ausgesät wurden, und für 20 min bei 37°C inkubiert. Fusion wurde durch Austausch der Fusionslösung gegen Zellkulturmedium gestoppt.

Im Vergleich dazu wurden 2 µg der identischen DNA ohne Komplexierung sowie ohne weitere Modifizierungen (kein Triton, kein Kation, kein zusätzlicher Ultraschallschritt) mit fusogenen Liposomen gleicher Konzentration und Zusammensetzung inkubiert und nachfolgend Zellen zur Fusion bei gleichen Bedingungen zur Verfügung gestellt (links). 24 Stunden nach Beendigung der Fusionsreaktion für DNA Übertragung sowie 3 Stunden für mRNA Übertragung wurde mittels Fluoreszenzmikroskopie und Durchflusszytometrie die Fusionseffizienz als auch die funktionelle Übertragung des Plasmids mittels Proteinexpressionsanalyse überprüft. Die in der Durchflusszytometrie als positiv identifizierten Zellen sind in Prozent angeben.

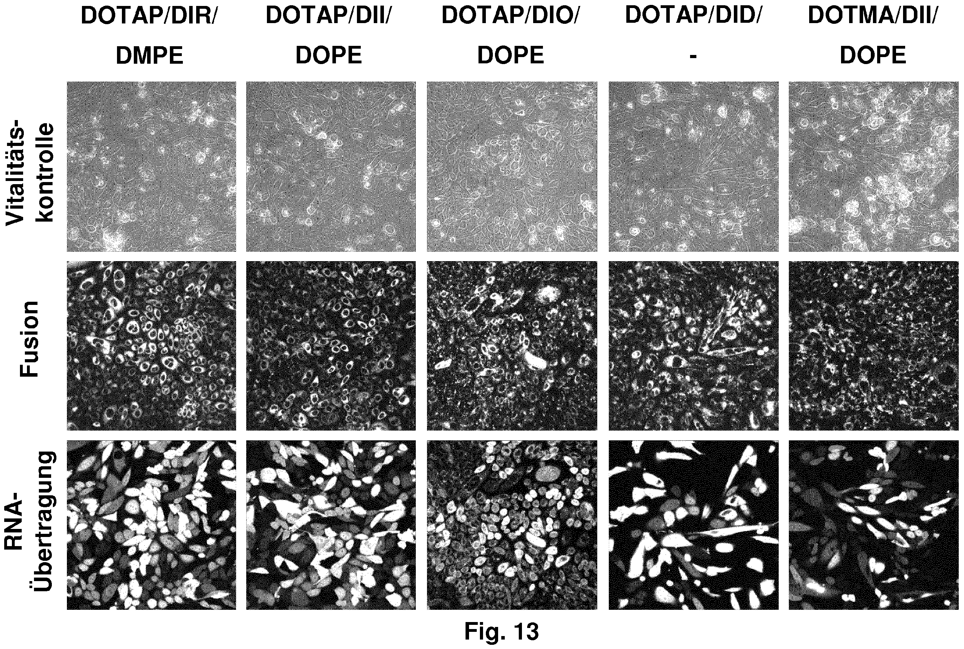

Fig. 13 zeigt die Verwendung unterschiedlicher Substanzen aus den Gruppen A, B und C zur Herstellung eines erfindungsgemäßen Fusionssystems. Zur Herstellung des Komplexes aus RNA und Protamin als Komplexbildner wurden 2 µg mRNA zur Expression von GFP (grün fluoreszierendes Protein) mit Protamin im Gewichtsverhältnis 2:1 inkubiert. Als Lösungspuffer wurden 5 µl Puffer verwendet (10 mM Tris, pH 7,5). Die Inkubation in Lösungspuffer erfolgte für 20 min bei RT. Während dieser Zeit wurden 10 µl einer 3 mM fusogenen Lipidmischung im Ultraschallbad bei 36 kHz und 200 W für 10 min bei RT suspendiert, um fusogene Liposome mit einer mittleren Größe von etwa 340 nm zu erhalten.

Als Komponenten A, B und C der Lipidzusammensetzung der fusogenen Liposomen wurden die in der Figur genannten Stoffe verwendet. Das Mischungsverhältnis der Komponenten A:B:C wurde für alle Ansätze mit einem Gewichtsverhältnis von 1:0,1:1 immer gleich gehalten. Nur der Ansatz mit fehlender Komponente C (DOTAP/DiD/-) hatte das Verhältnis 1:0,2.

Mischungen wurden in 20 mM HEPES, pH 7,4 angesetzt. Nach Beendigung der Inkubationszeiten wurden fusogene Liposome mit 1 µl einer 100 mM NaCl und 0,5 µM Triton X-100 Lösung versetzt und mit fertigem Komplex A für 20 min bei 36 kHz und RT im Ultraschallbad inkubiert. 10 µl der entstehenden fusogenen Komplex-A-Liposomen wurden mit PBS im Verhältnis 1:50 verdünnt und nochmals für 5 min bei gleichen Parametern im Ultraschallbad behandelt.

Die verdünnten Komplex-A-Liposomen wurden nachfolgend anstelle des Zellkulturmediums zu adhärenten CHO (Chinese hamster ovary) Zellen gegeben, die zuvor in einer Dichte von 15.000 Zellen pro cm² ausgesät wurden, und für 20 min bei 37°C inkubiert. Fusion wurde durch Austausch der Fusionslösung gegen Zellkulturmedium gestoppt.

3 Stunden nach Beendigung der Fusionsreaktion mit mRNA wurde mittels Fluoreszenzmikroskopie die Fusionseffizienz als auch die funktionelle Übertragung des mRNA mittels Proteinexpressionsanalyse überprüft. Alle Zusammensetzungen zeigten eine sehr hohe Fusionseffizienz und gute Übertragung geladener Moleküle.

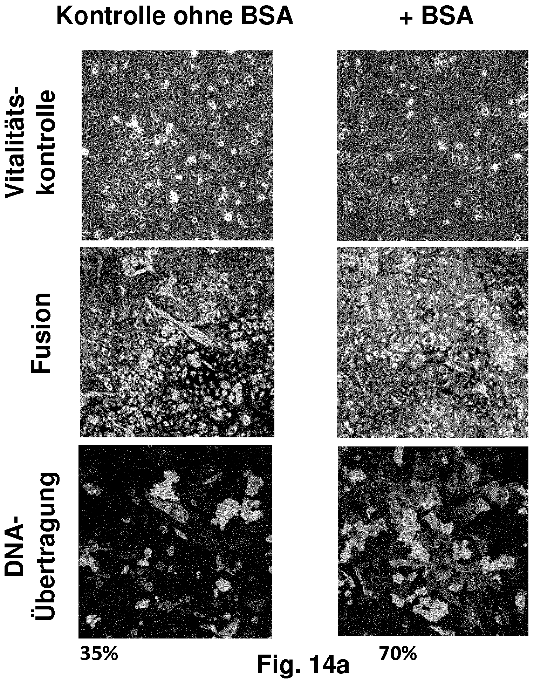

Fig. 14a illustriert den Einfluss von Albuminen auf die Übertragungseffizienz von geladenen Molekülen durch fusogene Liposomen. Zur Herstellung des Komplexes aus DNA und Komplexbildner (Komplex A) wurden 2 µg cDNA zur Expression von GFP (grün fluoreszierendes Protein) mit polykationischer Substanz (Protamin) im Gewichtsverhältnis 2:1 inkubiert. Als Lösungspuffer wurden 5 µl Puffer verwendet (10 mM Tris, pH 7,5). Diesem Puffer wurde 1 µl einer 0,5 mM Lösung von BSA (Bovines Serum Albumin) zugegeben. Die Inkubation in Lösungspuffer erfolgte für 5 min bei RT. Während dieser Zeit wurden 10 µl einer 3 mM fusogenen Lipidmischung im Ultraschallbad bei 46 kHz und 50 W für 10 min bei RT suspendiert, um fusogene Liposome mit einer mittleren Größe von etwa 340 nm zu erhalten.

Die Lipidzusammensetzung der fusogenen Liposome bestand aus positiv geladenem Lipid (DOTAP), fusogenem Molekül (DiR) und neutralem Lipid (DOPE) im Gewichtsverhältnis 1:0,1:1 in 20 mM HEPES, pH 7,4. Nach Beendigung der Inkubationszeiten wurden fusogene Liposome mit 1 µl einer 1 mM NaCl und 0,5 µM Triton X-100 Lösung versetzt und mit fertigem Komplex A für 10 min bei 46 kHz (50 W) und RT im Ultraschallbad inkubiert. 10 µl der entstehenden fusogenen Komplex-A-Liposomen wurden mit PBS im Verhältnis 1:50 verdünnt und nochmals für 5 min bei gleichen Parametern im Ultraschallbad behandelt.

Die verdünnten Komplex-A-Liposomen wurden nachfolgend anstelle des Zellkulturmediums zu adhärenten CHO (Chinese hamster ovary) Zellen gegeben, die zuvor in einer Dichte von 15.000 Zellen pro cm² ausgesät wurden, und für 20 min bei 37°C inkubiert. Fusion wurde durch Austausch der Fusionslösung gegen Zellkulturmedium gestoppt. 24 Stunden nach Beendigung der Fusionsreaktion wurde mittels Fluoreszenzmikroskopie und Durchflusszytometrie die Fusionseffizienz als auch die funktionelle Übertragung des Plasmids mittels Proteinexpressionsanalyse überprüft. Die in der Durchflusszytometrie als positiv identifizierten Zellen sind in Prozent angeben.

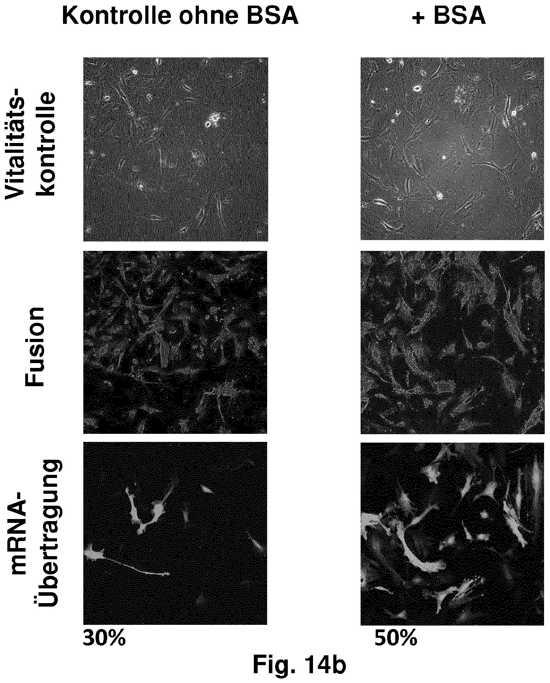

Fig. 14b illustriert den Einfluss von Albuminen auf die Übertragungseffizienz von geladenen Molekülen durch fusogene Liposomen. Zur Herstellung des Komplexes aus mRNA und Komplexbildner (Komplex A) wurden 2 µg mRNA zur Expression von GFP (grün fluoreszierendes Protein) mit polykationischer Substanz (Protamin) im Gewichtsverhältnis 2:1 inkubiert. Als Lösungspuffer wurden 5 µl Puffer verwendet (10 mM Tris, pH 7,5). Diesem Puffer wurde 3 µl einer 0,5 mM Lösung von HSA (Humanes Serum Albumin) zugegeben. Die Inkubation in Lösungspuffer erfolgte für 5 min bei RT. Während dieser Zeit wurden 10 µl einer 3 mM fusogenen Lipidmischung im Ultraschallbad bei 46 kHz und 50 W für 10 min bei RT suspendiert, um fusogene Liposome mit einer mittleren Größe von etwa 340 nm zu erhalten.

Die Lipidzusammensetzung der fusogenen Liposome bestand aus positiv geladenem Lipid (DOTAP), fusogenem Molekül (DiR) und neutralem Lipid (DOPE) im Gewichtsverhältnis 1:0,1:1 in 20 mM HEPES, pH 7,4. Nach Beendigung der Inkubationszeiten wurden fusogene Liposome mit 1 µl einer 1 mM NaCl und 0,5 µM Triton X-100 Lösung versetzt und mit fertigem Komplex A für 10 min bei 46 kHz (50 W) und RT im Ultraschallbad inkubiert. 10 µl der entstehenden fusogenen Komplex-A-Liposomen wurden mit PBS im Verhältnis 1:50 verdünnt und nochmals für 5 min bei gleichen Parametern im Ultraschallbad behandelt.

Die verdünnten Komplex-A-Liposomen wurden nachfolgend anstelle des Zellkulturmediums zu schwer zu fusionierenden, ausdifferenzierten, primären Myofibroblasten der Maus gegeben, die zuvor in einer Dichte von 15.000 Zellen pro cm² ausgesät wurden, und für 20 min bei 37°C inkubiert. Fusion wurde durch Austausch der Fusionslösung gegen Zellkulturmedium gestoppt. 3 Stunden nach Beendigung der Fusionsreaktion wurde mittels Fluoreszenzmikroskopie und Durchflusszytometrie die Fusionseffizienz als auch die funktionelle Übertragung des Plasmids mittels Proteinexpressionsanalyse überprüft. Die in der Durchflusszytometrie als positiv identifizierten Zellen sind in Prozent angeben.

Aus der Vielzahl unterschiedlicher Ausführungsbeispiele ergibt sich, dass das erfindungsgemäße Verfahren eine Fusionsmischung ergibt, die in zuverlässiger Weise eine Übertragung mittels Fusion in und/oder durch eine Lipidmembran ermöglicht, bei der insbesondere die übertragenen Moleküle ihre Funktion beibehalten.

## Patentansprüche

1. Verfahren zum Herstellen einer Fusionsmischung für eine Übertragung eines geladenen Moleküls in und/oder durch eine Lipidmembran mit den Schritten:
a) Bereitstellen einer Ausgangsmischung umfassend ein positiv geladenes amphipathisches Molekül A, ein aromatisches Molekül B mit hydrophobem Bereich und ein neutrales, amphipathisches Molekül C, wobei die Molekülsorten in einem Verhältnis A:B:C von 1 ― 2 : 0,02 ― 1 : 0 ― 1 mol/mol vorliegen,
b) Erzeugen eines fusogenen Liposoms durch Aufnahme der Ausgangsmischung in einem wässrigen Lösungsmittel,
c) Bereitstellen eines geladenen Moleküls,
d) Ausbilden eines Komplexes aus dem geladenen Molekül und einem neutralisierenden Agens,
e) Inkubieren des Komplexes mit dem fusogenen Liposom, so dass eine Fusionsmischung erhalten wird,
wobei Schritt d) derart erfolgt, dass der Komplex ein Zeta-Potential von -50 mV bis 0 mV aufweist,
wobei das geladene Molekül DNA oder RNA ist.

2. Verfahren nach einem der vorangegangenen Ansprüche, wobei vor Schritt e) ein Hinzufügen von Kationen erfolgt, um den Komplex zu stabilisieren.

3. Verfahren nach Anspruch 2, wobei die Kationen mit einer Konzentration von 0 bis 1 mM hinzugefügt werden.

4. Verfahren nach einem der vorangegangenen Ansprüche, wobei Schritt d) ein Hinzufügen von Albumin umfasst.

5. Verfahren nach einem der vorangegangenen Ansprüche, wobei vor, während und/oder nach Schritt e) ein Lipidmembran-destabilisierendes Agens hinzugefügt wird.

6. Verfahren nach Anspruch 5, wobei das Lipidmembran-destabilisierende Agens ein Detergens, insbesondere mit einen Kopf-zu-Ketten-Querschnittsverhältnis von wenigstens 1:1, ist.

7. Verfahren nach einem der vorangegangenen Ansprüche, wobei Schritt b) und/oder Schritt e) ein Durchführen einer Ultraschallbehandlung und/oder ein Durchführen einer Hochdruck-Homogenisierung umfasst.

8. Verfahren nach einem der vorangegangenen Ansprüche, wobei nach Schritt e) ein Verdünnen mit einem Puffer mit einer Osmolarität von 200 mOsm oder mehr erfolgen erfolgt.

9. Verfahren nach Anspruch 8, wobei der Puffer einen pH-Wert von 5 bis 10 aufweist.

10. Verfahren nach einem der Ansprüche 8 oder 9, wobei während des Verdünnens und/oder nach dem Verdünnen eine Ultraschallbehandlung und/oder eine Druckbeaufschlagung durchgeführt werden.

11. Verfahren nach einem der vorangegangen Ansprüche, wobei das Molekül A und/oder das Molekül C ein Lipid oder ein Lipidanalogon sind.

12. Verfahren nach einem der vorangegangenen Ansprüche, wobei das Molekül A und/oder das Molekül C bei Kontakt mit Wasser einen planaren oder nahezu planaren Bilayer ausbilden.

13. Fusionsmischung erhältlich nach einem der vorangegangenen Verfahren.

14. Verwendung einer Fusionsmischung nach Anspruch 13 zur Fusion mit einer Lipidmembran, insbesondere wobei die Lipidmembran eine Zellmembran oder ein Bestandteil einer Zellmembran ist.

15. Verfahren zur Übertragung eines geladenen Moleküls in und/oder durch eine Lipidmembran mittels Fusion, mit den Schritten:
Durchführen eines Verfahrens nach einem der Ansprüche 1―12,
In-Kontakt-Bringen der Fusionsmischung mit einer Lipidmembran, sodass die Fusionsmischung mit der Lipidmembran fusioniert,
wobei der Schritt des In-Kontakt-Bringens in Suspension und/oder mit einer auf einer Substratoberfläche adhärierten Lipidmembran durchgeführt wird.

16. Verfahren nach Anspruch 15, wobei die Lipidmembran eine Zellmembran oder ein Bestandteil einer Zellmembran ist.

## Claims

1. Method for producing a fusion mixture for a transfer of a charged molecule into and/or through a lipid membrane with the steps:
a) providing an initial mixture comprising a positively charged amphipathic molecule A, an aromatic molecule B with hydrophobic domain and a neutral, amphipathic molecule C, whereby the molecule types are at hand in a ratio A:B:C of 1 - 2 : 0.02 - 1 : 0 - 1 mol/mol,
b) generating a fusogenic liposome by inclusion of the initial mixture in a watery solvent,
c) providing a charged molecule,
d) forming a complex from the charged molecule and a neutralizing agent,
e) incubating the complex with the fusogenic liposome so that a fusion mixture is obtained,
wherein step d) occurs in a manner that the complex has a zeta potential of -50 mV to 0 mV,
wherein the charged molecule is DNA or RNA.

2. Method according to one of the preceding claims, wherein before step e) an adding of cations occurs in order to stabilize the complex.

3. Method according to claim 2, wherein the cations are added with a concentration of 0 to 1 mM.

4. Method according to one of the preceding claims, wherein step d) comprises an adding of albumin.

5. Method according to one of the preceding claims, wherein before, during and/or after step e) a lipid membrane destabilizing agent is added.

6. Method according to claim 5, wherein the lipid membrane destabilizing agent is a detergent, in particular with a head-to-chain aspect ratio of at least 1:1.

7. Method according to one of the preceding claims, wherein step b) and/or step e) comprise an implementation of an ultrasonic treatment and/or an implementation of a high-pressure homogenization.

8. Method according to one of the preceding claims, wherein after step e), a dilution with a buffer with an osmolarity of 200 mOsm or more occurs.

9. Method according to claim 8 wherein the buffer has a pH-value of 5 to 10.

10. Method according to one of the claims 8 or 9, wherein during the dilution and/or after the dilution, an ultrasonic treatment and/or a pressurization is carried out.

11. Method according to one of the preceding claims, wherein molecule A and/or molecule C are a lipid or a lipid analogon.

12. Method according to one of the preceding claims, wherein molecule A and/or molecule C, at contact with water generate a planar or nearly planer bilayer.

13. Fusion mixture obtainable according to one of the preceding methods.

14. Use of a fusion mixture according to claim 13 for fusion of a lipid membrane, in particular wherein the lipid membrane is a cell membrane or a compound of a cell membrane.

15. Method for transfer of a charged molecule in and/or through a lipid membrane by means of fusion, with the steps:
performing a method according to one of the claims 1 to 12,
bringing into contact the fusion mixture with a lipid membrane so that the fusion mixture fuses with the lipid membrane,
wherein the step of bringing into contact is performed in suspension and/or with a lipid membrane adhered on a substrate surface.

16. Method according to claim 15, wherein the lipid membrane is a cell membrane or a compound of a cell membrane.

## Revendications

1. Procédé de fabrication d'un mélange de fusion pour le transfert d'une molécule chargée dans et/ou à travers une membrane lipidique, comportant les étapes suivantes :
a) préparation d'un mélange initial comportant une molécule amphiphile chargée positivement A, une molécule aromatique B avec un groupe hydrophobe, et une molécule amphiphile neutre C, dans lequel la distribution des différentes molécules répond à une proportion A:B:C de 1-2 : 0,02-1 : 0-1 mol/mol,
b) production d'un liposome fusogène par absorption du mélange initial dans un solvant aqueux,
c) préparation d'une molécule chargée,
d) formation d'un complexe à partir de la molécule chargée et d'un agent neutralisant,
e) incubation du complexe avec le liposome fusogène, de manière à obtenir un mélange de fusion,
dans lequel l'étape d) se produit de telle sorte que le complexe présente un potentiel zêta de -50 mV à 0 mV,
dans lequel la molécule chargée est une molécule d'ADN ou d'ARN.

2. Procédé selon l'une des revendications précédentes, dans lequel avant l'étape e) se produit l'ajout de cations pour stabiliser le complexe.

3. Procédé selon la revendication 2, dans lequel les cations sont ajoutés avec une concentration de 0 mM à 1 mM.

4. Procédé selon l'une des revendications précédentes, dans lequel l'étape d) comprend l'ajout d'albumine.

5. Procédé selon l'une des revendications précédentes, dans lequel avant, pendant et/ou après l'étape e), un agent de déstabilisation de la membrane lipidique est ajouté.

6. Procédé selon la revendication 5, dans lequel l'agent de déstabilisation de la membrane lipidique est un détergent, en particulier avec un rapport de forme tête/chaîne d'au moins 1:1.

7. Procédé selon l'une des revendications précédentes, dans lequel l'étape b) et/ou l'étape e) comprend la mise en œuvre d'un traitement par ultrason et/ou d'une homogénéisation à haute pression.

8. Procédé selon l'une des revendications précédentes, dans lequel après l'étape e), une dilution a lieu avec une solution tampon dont l'osmolarité est supérieure à égale à 200 mOsm.

9. Procédé selon la revendication 8, dans lequel le pH de la solution tampon est compris entre 5 et 10.

10. Procédé selon l'une des revendications 8 et 9 dans lequel, durant et/ou après la dilution, un traitement par ultrason et/ou une mise sous pression est exécuté.

11. Procédé selon l'une des revendications précédentes, dans lequel la molécule A et/ou la molécule C est un lipide ou un analogue de lipide.

12. Procédé selon l'une des revendications précédentes, dans lequel la molécule A et/ou la molécule C forment une bicouche plane ou pratiquement plane lorsqu'elles sont mises en contact avec de l'eau.

13. Mélange de fusion obtenu grâce à l'un des procédés précédents.

14. Utilisation d'un mélange de fusion selon la revendication 13 pour une fusion avec une membrane lipidique, en particulier dans laquelle la membrane lipidique est une membrane cellulaire ou un composant d'une membrane cellulaire.

15. Procédé de transfert d'une molécule chargée par fusion dans et/ou à travers une membrane lipidique, comprenant les étapes suivantes :
mise en œuvre d'un procédé selon l'une des revendications 1 à 12,
mise en contact du mélange de fusion avec une membrane lipidique, de telle sorte que le mélange de fusion fusionne avec la membrane lipidique,
dans lequel l'étape de mise en contact est mise en œuvre en suspension et/ou avec une membrane lipidique adhérant à une surface de substrat.

16. Procédé selon la revendication 15, dans lequel la membrane lipidique est une membrane cellulaire ou un composant d'une membrane cellulaire.
